# EUROPEAN PATENT SPECIFICATION

(11) **EP 1 816 968 B1**
(45) Date of publication and mention of the grant of the patent: **31.03.2010**
(21) Application number: 05810819.2
(22) Date of filing: 01.12.2005
(51) Int. Cl.: A61B 17/34, A61B 17/02

(54) **A SURGICAL SEALING DEVICE**
CHIRURGISCHE VERSCHLUSSVORRICHTUNG
DISPOSITIF DE FERMETURE CHIRURGICAL

(30) Priority: 01.12.2004 US 631976 P
(43) Date of publication of application: 15.08.2007
(73) Proprietor: Atropos Limited, Bray County Wicklow (IE)
(72) Inventor: BUTLER, John, Blackrock, County Dublin (IE); VAUGH, Trevor, Birr, County Offaly (IE); BONADIO,Frank, Bray, County Dublin (IE)
(74) Representative: O'Brien, John Augustine
(86) International application number: PCT/IE2005/000135
(87) International publication number: WO 2006/059318

(56) References cited:
- WO-A-02/34108
- US-A- 5 514 133
- US-A- 6 033 426
- US-A1- 2005 148 823

## Description

### Introduction

This invention relates to a surgical sealing device suitable for use during a surgical procedure, especially suitable for use during laparoscopic surgery or during hand assisted laparoscopic surgery.

In carrying out a surgical procedure in the region of the abdomen, it is known to form an incision and then retract the sides of the incision to provide an access opening. The opening may be sealed with a valve in an effort to prevent insufflation gases from escaping and to maintain pneumoperitoneum.

US-A-5514133 describes a device of this type having a proximal seal member of silastic, foam or cellular material which seals and expands when a hand or an instrument is inserted through an opening in the seal member. In one case there are flap valve seals which are movable for passage of an object. US-A-6033426 also describes a device of this type having a first outer valve and a second inner valve. The first valve is of an elastically deformable thin film material. The second valve comprises a plurality of film bodies.

It is however difficult to provide an effective gas seal while providing access for an instrument or for a surgeon's hand.

This invention is directed towards providing a surgical sealing device, which will address these problems.

According to the invention there is provided a surgical sealing device comprising:
a first stealing valve for sealing across an opening to an internal cavity;

The invention relates to a surgical sealing device comprising:
a first scaling valve for sealing across an opening to an internal cavity;
the first sealing valve comprising a first sealing member and a second sealing member;
the sealing members being movable relative to one another between a closed configuration, in which the sealing members at least partially overlap one another, for sealing across the opening, and an open configuration for the first sealing valve comprising a first sealing member and a second sealing member;
the sealing members being movable relative to one another between a closed configuration, in which the sealing members at least partially overlap one another, for sealing across the opening, and an open configuration for facilitating passage of an object through the first sealing valve to access the internal cavity; and
at least one support element for at least partially supporting at least one of the sealing members to facilitate ease of passage of the object through the first sealing valve.

GB-A-2298906 and WO-A-03/0111153 describe trocar seal systems.

The invention is characterised in that the support element is attached to the exterior surface of the first sealing member or in that the support element is embedded within the first sealing member.

In one embodiment of the invention the support element is configured to at least partially support the sealing member to facilitate ease of movement of the sealing members relative to one another from the closed configuration to the open configuration. The support element may be configured to minimise deformation of at least part of the sealing member.

In one case the sealing member is substantially planar. The support element may be configured to minimise deformation of at least part of the sealing member in a direction substantially perpendicular to the plane of the sealing member.

The support element may be substantially planar,

In another embodiment the sealing members are fixedly attached to one another, each sealing member having a first region attached to other sealing member and a second region which remains detached from the other sealing member. The support element may be configured to minimise deformation of the second region of the sealing member.

In one case the support element.comprises a stiffening element to increase the stiffness of at least part of the sealing member.

In one embodiment of the invention at least one of the sealing member is at least partially of a gelatinous elastomeric material. In one case both sealing members are at least partially of a gelatinous elastomeric material. In another case the material comprises a plasticizer. The plasticizer may be selected from the group consisting of naturally derived oils, synthetic oils and liquid oligomers.

In one case the first sealing valve is biased towards the closed configuration. In one case at least one of the sealing members is of a resilient material to bias the first sealing valve towards the closed configuration.

The first sealing member and the second sealing member may define overlapping portions, which overlap with each other in the closed configuration. In one case at least the overlapping portions of the sealing members are generally planar. In another case the planes of the overlapping portions are slightly offset. In another case the overlapping portions engage with each other in the closed configuration. The engagement force is in one case overcome on insertion of an object, such as a surgeon's hand.

In another embodiment a thin slit is defined between the overlap of the first sealing member over the second sealing member.

The first sealing member may be fixedly attached to the second seating member. In one case the first sealing member is formed integrally with the second sealing member. In another case the first sealing member is moulded integrally with the second sealing member. In one case the first sealing member comprises an overlap region, which overlaps the second sealing member in the closed configuration, the overlap region comprising a first region attached to a second sealing member and a second region detached from the second sealing member. In one case a thin slit is defined between the second region of the first sealing member and the second sealing member. In another case the slit extends circumferentially through approximately 90°. The ratio of the radial dimension of the slit to the overall radial dimension of the entire first sealing member may be approximately 1:4, in one case approximately 1:8.

In a preferred case in the closed configuration, the second region of the first sealing member engages the second sealing member. In another case the second region of the first sealing member is biased towards engagement with the second sealing member.

The first sealing valve may be configured to be located externally of an internal cavity proximally of an opening to the internal cavity. The first sealing valve may be configured to be located within an internal cavity distally of an opening to the internal cavity.

The first sealing member is in one case substantially planar. The second sealing member is in one case substantially planar.

In a preferred embodiment the device comprises a second sealing valve for sealing around an object passed through an opening to an internal cavity.

The first sealing member may be located distally of the second sealing valve. The first sealing valve may be located proximally of the second sealing valve.

In one case the first sealing valve is longitudinally spaced apart from the second sealing valve. One sealing valve may be configured to be located externally of an internal cavity proximally of an opening to the internal cavity, and the other sealing valve may be configured to be located within the internal cavity distally of the opening.

In another case the first sealing valve is located adjacent to and in close proximity to the second sealing valve. Both the first sealing valve and the second sealing valve may be located externally of an internal cavity proximally of an opening to the internal cavity. Both the first sealing valve and the second sealing valve may be located within an internal cavity distally of an opening to the internal cavity.

In one case the second sealing member provides the second sealing valve. In another case the second sealing member has a passageway extending therethrough, through which an object may be inserted to access an internal cavity. The second sealing member may be configured to effect a seal between a sidewall of the passageway and an object inserted through the passageway.

In one case, in the closed configuration, the first sealing member extends across an end of the passageway and overlaps the second sealing member around the entire periphery of the end of the passageway. In one case in the open configuration the first sealing member is retracted to reveal at least part of the end of the passageway.

The second sealing valve may comprise a lip seal valve.

In a further embodiment the device is mountable to a retractor device. In one case the device comprises a mounting element for mounting the device to a retractor device. In another case the mounting element is fixedly attached to the sealing valve. In another case a part of the sealing valve is overmoulded around a part of the mounting element. The mounting element may comprise one or more overmould openings therein. In one case the mounting element comprises an engagement formation for snap-fit mounting of the device to a retractor device.

The mounting element may comprise a ring element.

In one case the device is configured to effect a seal between the device and a retractor device upon mounting of the device to the retractor device. In another case at least one of the sealing members is configured to engage a retractor device upon mounting.

In another embodiment the first sealing valve comprises a biasing member to bias the first sealing valve towards the closed configuration. In one case the biasing member comprises a resilient element extending between the first sealing member and the second sealing member. The resilient element may comprise a spring.

The biasing member may comprise a magnetic element on the first sealing member and a co-operating magnetic element on the second sealing member.

In another case the first sealing valve comprises a biasing member to bias the second region of the first sealing member towards engagement with the second sealing member. In one case the biasing member comprises a resilient element for tensioning the second region of the first sealing member. In another case the resilient element extends between the second region of the first sealing member and the second sealing member.

In one case the first sealing member is hingeably movable relative to the second sealing member between the closed configuration and the open configuration.

The first sealing member may be at least partially curved.

The first sealing member may be at least partially substantially spherical. The second sealing member may be substantially curved.

In the closed configuration, the first sealing member may extend at least partially into the passageway.

In another embodiment the thickness of the second sealing member varies across the width of the second sealing member. In one case the thickness of the second sealing member is at a maximum adjacent the passageway.

The passageway may define a longitudinal axis, and the passageway longitudinal axis may be configured to subtend an acute angle with a longitudinal axis defined by an opening to an internal cavity. In one case the longitudinal axis of the passageway is angled towards the second region of the first sealing member.

In another case the device comprises a closure member for closing the passageway. In one case the closure member comprises a plug.

In one embodiment the second sealing valve comprises a third sealing member having a passageway extending therethrough, through which an object may be inserted to access an internal cavity. In one case the third sealing member is configured to effect a seal between a wall of the passageway and an object inserted through the passageway. In another case the third sealing member is at least partially of a gelatinous elastomeric material.

The second sealing valve may be at least partially inflatable. The second sealing valve may comprise an iris valve.

In a further case the device comprises a third sealing valve for sealing across an opening to an internal cavity. In one case the third sealing valve comprises a fourth sealing member, the second sealing member and the fourth sealing member being movable relative to one another between the closed configuration, in which the second sealing member and the fourth sealing member at least partially overlap one another, and the open configuration. In another case the fourth sealing member is at least partially of a gelatinous elastomeric material.

The mounting element may comprise a screw-thread formation for screw-thread mounting of the device to a retractor device.

In one case the mounting element comprises a sleeve extending proximally of the sealing valve. In one case the sleeve is hookable around a part of a retractor device to mount the device to the retractor device. The sleeve may comprise a mounting ring for hooking around a part of a retractor device.

According to another aspect, the invention provides a surgical assembly comprising:
a retractor device for retracting the sides of an opening to an internal cavity; and
a surgical sealing device of the invention.

In one case the surgical sealing device is mounted to the retractor device.

The retractor device may have a distal end and a proximal end, and the surgical sealing device may be located adjacent to the distal end of the retractor device. The retractor device may have a distal end and a proximal end, and the surgical sealing device may be located adjacent to the proximal end of the retractor device.

### Brief Description of the Drawings

The invention will be more clearly understood from the following description of some embodiments thereof, given by way of example only, with reference to the accompanying drawings, in which: -
Fig. 1 is a perspective view from above of a surgical sealing device;
Fig. 2 is a perspective view from below of the device of Fig.1;
Fig. 3 is a plan view from above of the device of Fig. 1;
Fig. 4 is a side view in the direction of arrow A in Fig. 3;
Fig. 5 is a view along line V-V in Fig. 3;
Fig. 6 is a view along line VI-VI in Fig. 3;
Fig. 7 is a partially cross sectional, side view of the device of Fig. 1, in use;
Fig. 8 is a partially cross-sectional, side view of the device of Fig. 1 mounted to a retractor device;
Fig. 9 is a perspective view of a mounting element for the device of Fig. 1;
Fig. 10 is a cut-away, perspective view of the mounting element of Fig. 9;
Fig. 11 is a cross-sectional, side view of the device of Fig. 1 and the mounting element of Fig. 9;
Fig. 12 is a cut-away, perspective view of the device of Fig. 1 and the mounting element of Fig. 9;
Fig. 13 is a cross-sectional, side view of the device of Fig. 1 and the mounting element of Fig. 9 mounted to a retractor device;
Fig. 14 is a perspective view illustrating mounting of the device of Fig. 1 and another mounting element to another retractor device;
Fig. 15 is a cross-sectional, side view of another surgical scaling device;
Fig. 16 is a perspective view of a part of the device of Fig. 15;
Figs. 17 to 21 are cross-sectional, side views of other surgical sealing devices;
Figs. 22 and 23 are plan views from above of further surgical sealing devices;
Figs. 24 and 25 are perspective views of another surgical sealing device;
Fig. 26 is a cross-sectional, side view of the device of Fig. 25;
Fig. 27 is a perspective view of another surgical sealing device;
Fig. 28 is a perspective view of a further surgical sealing, device;
Fig. 29 is a side view of the device of Fig. 28;
Fig. 30 is a plan view of the device of Figs. 28 and 29 with an object, such as a surgeon's arm, in position;
Fig. 31 is a perspective view of another surgical sealing device;
Fig. 32 is plan view of the device of Fig. 31;
Fig. 33 is a cross sectional, side view of the device of Fig. 31;
Figs. 34 and 35 are partially cross sectional, side views illustrating the device of Figs. 31 to 33, in use;
Figs. 36 and 37 are cross sectional, side views of other surgical sealing devices;
Fig. 38 is a perspective view of a lip seal used in some aspects of the invention;
Figs. 39 and 40 are cross sectional, side views of another surgical sealing device incorporating a distal overlap valve and a proximal lip seal;
Fig. 41 is a partially cross sectional, side view of the device of Figs. 39 and 40, in use;
Fig. 42 is a cross sectional, side view of another surgical sealing device mounted to a retractor device;
Fig. 43 is a partially cross sectional, side view of the device of Fig. 42, in use;
Fig. 44 is a cross sectional, side view of another surgical sealing device with a distal overlap valve and a lip seal located proximally adjacent to the overlap valve;
Fig. 45 is a partially cross sectional, side view of the device of Fig. 44, in use;
Fig. 46 is a cross sectional, side view of another surgical sealing device with a lip seal and an overlap valve located proximally of a retractor device;
Fig. 47 is a cross sectional, side view of another surgical sealing device with an overlap valve incorporating a lip seal;
Fig. 48 is a partially cross sectional, side view of the device of Fig. 47, in use;
Fig. 49 is a cross sectional, side view of a further surgical sealing device incorporating a secondary valve;
Figs. 50 to 53 are partially cross sectional, side views of the device of Fig. 49, in use;
Fig. 54 is a plan view of the secondary valve of the device of Fig. 49 in the configuration of Fig. 53;
Fig. 55 is a cross sectional, side view of another surgical sealing device mounted to a retractor device;
Fig. 56 is a partially cross sectional, side view of the device of Fig. 55, in use;
Fig. 57 is a cross sectional, side view of another surgical scaling device in one configuration of use;
Fig. 58 is a cross sectional, side view of the device of Fig. 57 in another configuration of use;
Fig. 59 is a partially cross sectional, side view of the device of Figs. 57 and 58 with a hand inserted;
Fig. 60 is a cross sectional, side view of another surgical sealing device mounted to a retractor device;
Fig. 61 is a cross sectional, side view of another surgical sealing device mounted to a retractor device;
Fig. 62 is a perspective view of a secondary valve of the device of Fig. 61;
Fig. 63 is an exploded, perspective view of another surgical sealing device;
Fig. 64 is a cross-sectional, side view of the device of Fig. 63;
Figs. 65 to 67 are perspective views from below of further surgical sealing devices;
Fig. 68 is a plan view from below of the device of Fig. 67;
Fig. 69 is a cross-sectional, side view of another surgical sealing device;
Fig. 70 is a partially cross-sectional, side view of the device of Fig. 69, in use;
Fig. 71 is a partially cross-sectional, side view of a further surgical sealing device;
Fig. 72 is a perspective view from below of the device of Fig. 71;
Fig. 73 is a partially cross-sectional, side view of another surgical sealing device;
Fig. 74 is a partially cross-sectional, side view of the device of Fig. 73, in use;
Fig. 75 is a perspective view from below of a surgical sealing, device according to the invention;
Figs. 76 and 77 are cross-sectional, side views of the device of Fig. 75;
Fig. 78 is a partially cross-sectional, side view of the device of Fig. 75, in use; and
Figs. 79 and 80 are views similar to Figs. 75 and 76 of a further surgical sealing device according to the invention.

### Detailed Description

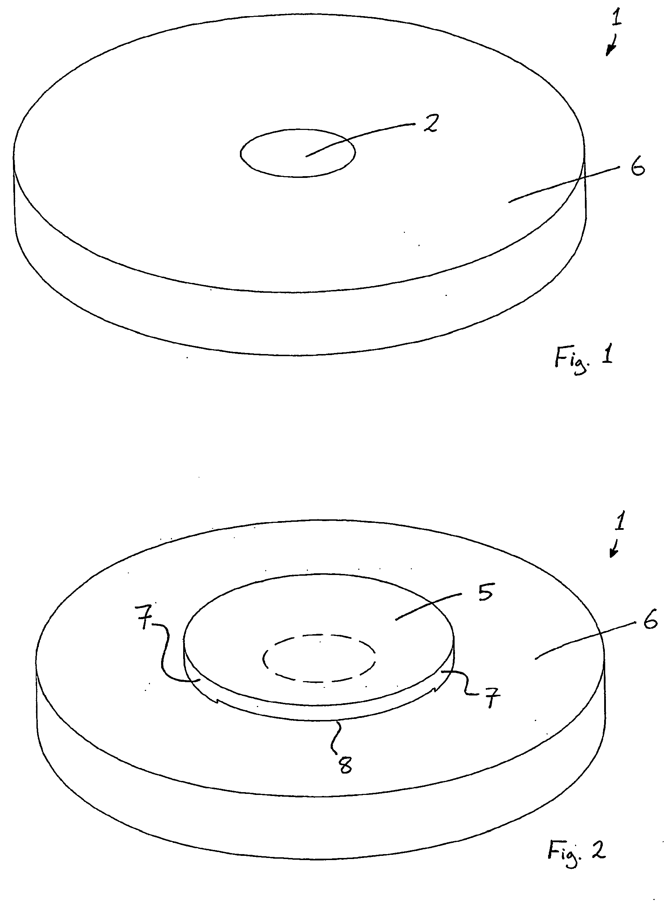

Referring to the drawings and initially to Figs. 1 to 8 thereof, there is illustrated a surgical sealing device 1, which is suitable for use during
a surgical procedure, such as a laparoscopic procedure or a hand-assisted laparoscopic procedure.

The device 1 may in the case of a laparoscopic procedure be employed to effect a seal around a laparoscopic instrument inserted through the device 1 into an abdomen to maintain insufflation gas pressure within the abdomen. In the case of a hand-assisted laparoscopic procedure, the device 1 may be employed to effect a seal around a surgeon's hand or forearm inserted through the device 1 into the abdomen to maintain insufflation gas pressure within the abdomen.

The device 1 comprises a planar first sealing member 5 and a planar second sealing member 6. The second sealing member 6 has a passageway 2 extending therethrough through which an object, such as a laparoscopic instrument or a surgeon's hand / forearm 3, may be inserted to access an interior space, such as an insufflated abdomen 4. The planar first sealing member 5 may be employed to seal the passageway 2.

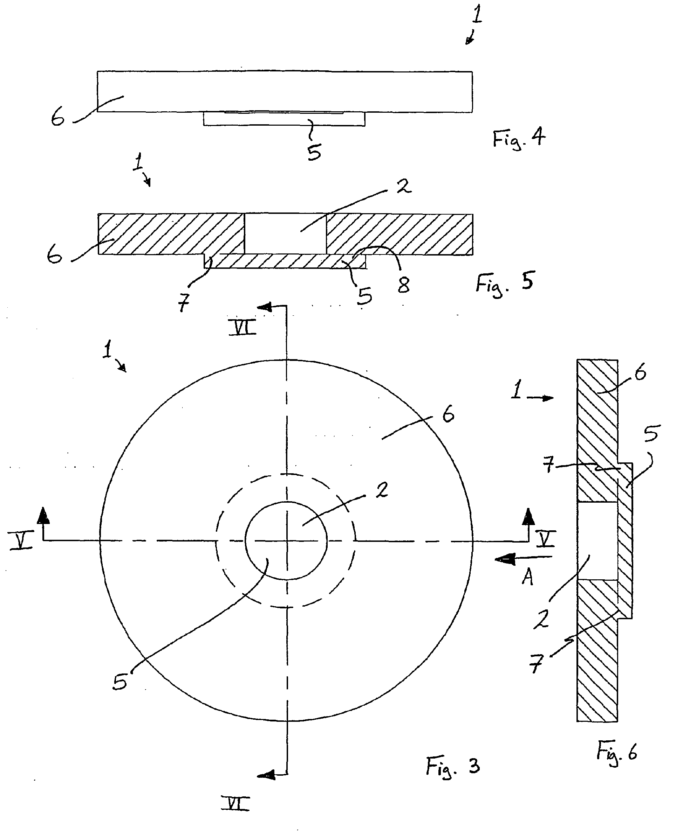

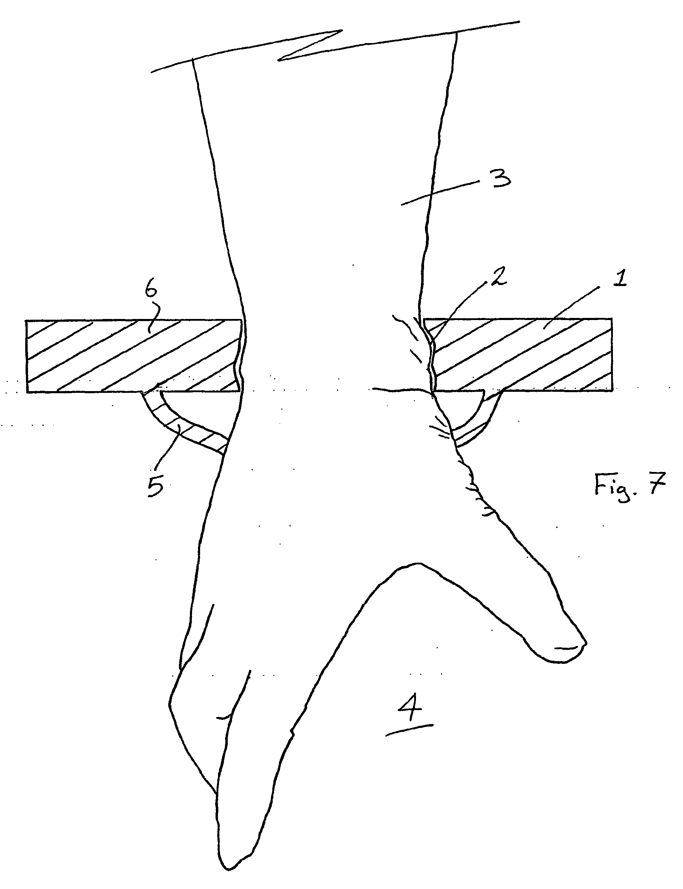

In this case, the first sealing member 5 is provided in the form of a flap member of an elastic material. The flap member 5 is movable relative to the second sealing member 6 between a closed configuration sealing the passageway 2 (Fig. 5), and an open configuration to facilitate insertion of an object, such as the surgeon's forearm 3 through the passageway 2 to access the abdomen 4 (Fig. 7). In the closed configuration, the flap member 5 extends across the distal end of the passageway 2 and overlaps the entire periphery of the distal end of the passageway 2 to prevent leakage of the insufflation gas out of the abdomen 4. The insufflation gas pressure within the abdomen 4 forces the flap member 5 into engagement with the second sealing member 6 in the closed configuration. In the open configuration, the flap member 5 is retracted to reveal the distal end of the passageway 2 to enable an object, such as the surgeon's hand / forearm 3 to pass through the sealing device 1 and into the abdomen 4. In this manner the flap member 5 defines an overlap seal.

Insertion of the object, such as the hand/forearm 3 through the passageway 2 pushes against the flap member 5 to cause retraction of the flap member 5.

In this case the first sealing member 5 and the second sealing member 6 are formed integrally, in this case moulded integrally. In the closed configuration, the first sealing member 5 overlaps the second sealing member 6 around the entire periphery of the distal end opening of the passageway 2. In this region of overlap, the first sealing member 5 is fixedly attached to the second sealing member 6 at an attachment region 7, and is detached from the second sealing member 6 at a detachment region 8. As illustrated in Figs. 2 to 6, the detachment region 8 is in the form of a very thin slit or gap. The slip / gap 8 extends circumferentially through approximately 90°, and has a radial dimension approximately ¼ of the radial width of the first sealing member 5, and approximately 1/8 of the radial width of the second sealing member 6. In the closed configuration, the insufflation gas pressure within the abdomen 4 forces the detachment region 8 to engage against the second sealing member 6 around the distal end of the passageway 2. This results in the thin slit / gap being closed down to prevent leakage of insufflation gas out of the abdomen 4.

Because the region 8 is detached from the second sealing member 6, this arrangement enables the region 8 to be retracted when an object is inserted through the passageway 2, which results in the slit / gap becoming large enough for the object to exit the passageway 2 and access the abdomen 4. As the object passes through the passageway 2, the object engages the region 8 and forces the region 8 aside. In this manner, the region 8 is retracted.

Because the first sealing member 5 and the second sealing member 6 overlap in the closed configuration, a seal is created across the wound opening which prevents loss of insufflation gases from the abdomen 4. The overlapping first and second sealing members 5, 6 thus provide a first sealing valve for sealing across the wound opening.

The elastic biasing nature of the flap member 5 biases the flap member 5 towards the closed configuration to prevent leakage of insufflation gas out of the abdomen 4, when the surgeon withdraws the object, such as the hand / forearm 3 out of the abdomen 4. In particular the resilience of the material of the flap member 5 biases the flap member 5 towards the closed configuration. In addition, the gas pressure within the abdomen 4 acts on the flap member 5 to force the flap member 5 towards the closed configuration.

The sidewall of the passageway 2 is of an elastic material, and the passageway 2 typically has a smaller diameter than an object to be inserted therethrough. The material defining the passageway 2 thus creates a seal between the sidewall of the passageway 2 and the object, defining an interference fit between the sidewall and the object (Fig. 7). In this manner, the passageway 2 through the second sealing member 6 acts as a second sealing valve to seal around the object while the object is inserted through the passageway 2 and into the abdomen 4. In particular the passageway 2 acts as a lip seal. Thus leakage of insufflation gas out of the abdomen 4 while the object is inserted through the passageway 2 and into the abdomen 4 is prevented.

In the case of laparoscopic surgery, the passageway 2 is sized to seal around a laparoscopic instrument inserted therethrough. The passageway 2 may therefore be sized as a pinhole through the second sealing member 6. In certain cases, the second sealing member 6 may be formed with no hole therethrough, and the passageway 2 may be created in-situ by the surgeon piercing the second sealing member 6 with the instrument and forcing the instrument through the second sealing member 6.

In the case of hand-assisted laparoscopic surgery, the passageway 2 is sized to seal around a hand or forearm of the surgeon.

As illustrated in Fig. 7, the first sealing overlap valve provided by the flap member 5 is located longitudinally distally of the second sealing lip seal valve provided by the passageway 2.

However it will be appreciated that the first sealing valve may alternatively be provided proximally of the second sealing valve.

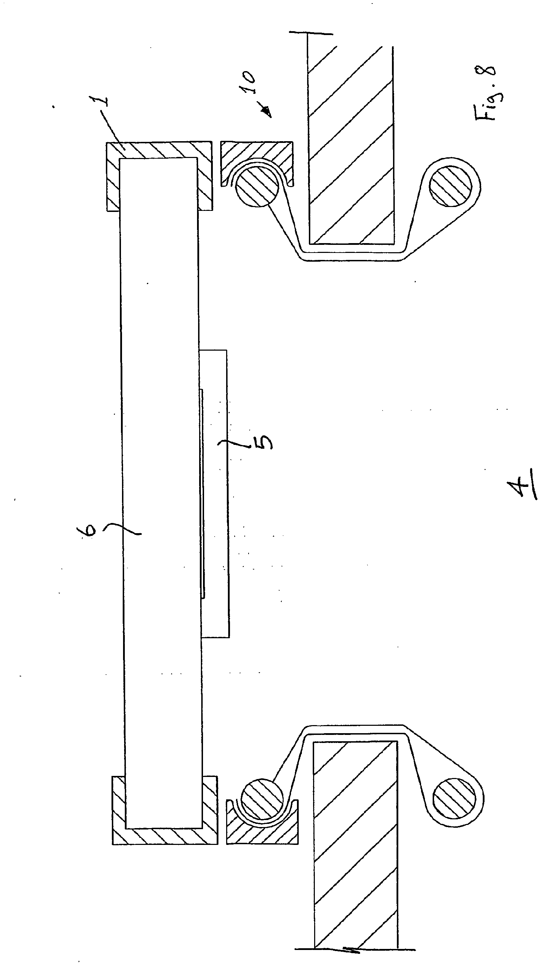

The sealing device 1 may be mounted to a wound retractor device 10, as illustrated in Fig. 8. The mounting may be in any convenient manner such as a releasable mounting using a bayonet type mounting or the like as will be described in more detail below. The gas pressure within the abdomen 4, after insufflation, pushes up on the first sealing member 5, causing the first sealing member 5 to engage against the second sealing member 6 to maintain pneumoperitoneum.

The device 1 is in this case a combined overlap/lipseal valve for hand assisted laparoscopy (HALS).

The device 1 may be a single piece moulded gel component. The first and second sealing members 5, 6 are both formed of a gel material. The gel material may include an elastomer, such as silicone or latex. The gel material may also include an oil, and/or a foam.

In one embodiment the device 1 is of a gelatinous elastomeric material. An extensive review of gelatinous elastomeric materials is included in US 5,994,450 (Pierce), the entire contents of which are incorporated herein by reference. One such group of gelatinous elastomers may comprise a triblock copolymer A-B-A wherein A is selected from the group consisting of monoalkenylarene polymers and B is a hydrogenated polymer including a plurality of isoprene monomers and a plurality of butadiene monomers. The material includes a plasticiser which may be selected from the group consisting of naturally derived oils, synthetic oils and liquid oligomers. For the device of this embodiment of the invention the gelatinous elastomeric material is formulated to have high tear strength and high flexibility.

The materials required to form a suitable gel material are available, for example, from the company Edizone L.C. of Utah, USA. The gel material preferably has the properties of high tear strength to resist tearing of the attachment of the first sealing member 5 to the second sealing member 6, and high flexibility to enable the first sealing member 5 to be retracted for passage of an object through the passageway 2 into the abdomen 4.

During moulding, a thin plate is located between the first sealing member 5 and the second staling member 6. This thin plate prevents the first sealing member 5 being fully moulded to the second sealing member 6. As a result the first sealing member 5 is moulded to the second sealing member 6 at a first region which is the attachment region 7, and remains un-moulded to the second sealing member 6 at a second region which is the detachment region 8. This thin plate defines the thin slit / gap 8 between the sealing members 5, 6.

In certain applications the device may alternatively be formed as a single piece moulded rubber or elastomeric component.

There is a lipseal hole 2 through which an object, such as hand can pass, due to the highly elastic nature of the material. An overlap flap 5 is integrally moulded underneath, and covering, the lipseal hole 2. The overlap flap 5 is essentially joined to/integral with the main body of the lipseal apart from a slit which in this case extends radially. Because of the elastic nature of the material, the object, such as the hand can displace the overlap flap 5 and push through the slit. In use, the slit is closed by the gas pressure acting upwardly on the flap 5. On insertion of an object the slit can be opened just sufficiently to allow the object to pass therethrough with no substantial loss of gas pressure. The lipseal 2 now seals to the object, such as the surgeon's wrist, maintaining pneumoperitoneum. When the object, such as the hand/forearm 3 is removed, the overlap flap 5 returns to its original position blocking the lipseal hole 2.

In the case where the surgical sealing device of the invention is employed during laparoscopic surgery, the second sealing member 6 may alternatively be formed as a continuous single block of gel with no passageway extending therethrough. When the device is mounted across a wound opening sealing the opening, the surgeon may pierce the second sealing member 6, for example with a laparoscopic instrument having a sharp distal tip to create the passageway through the second sealing member 6 in situ by pushing the instrument through the second sealing member 6.

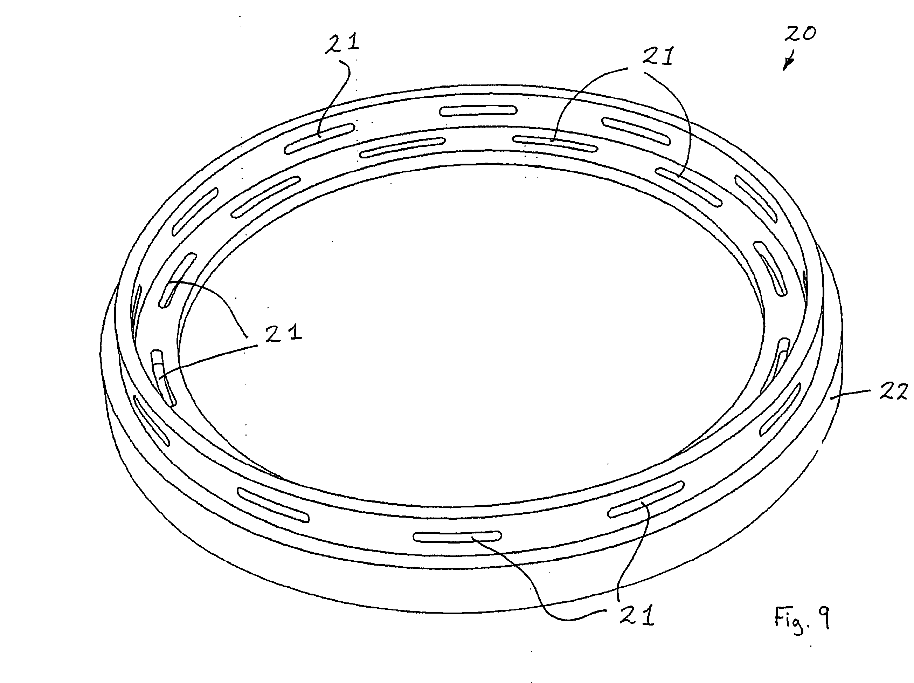

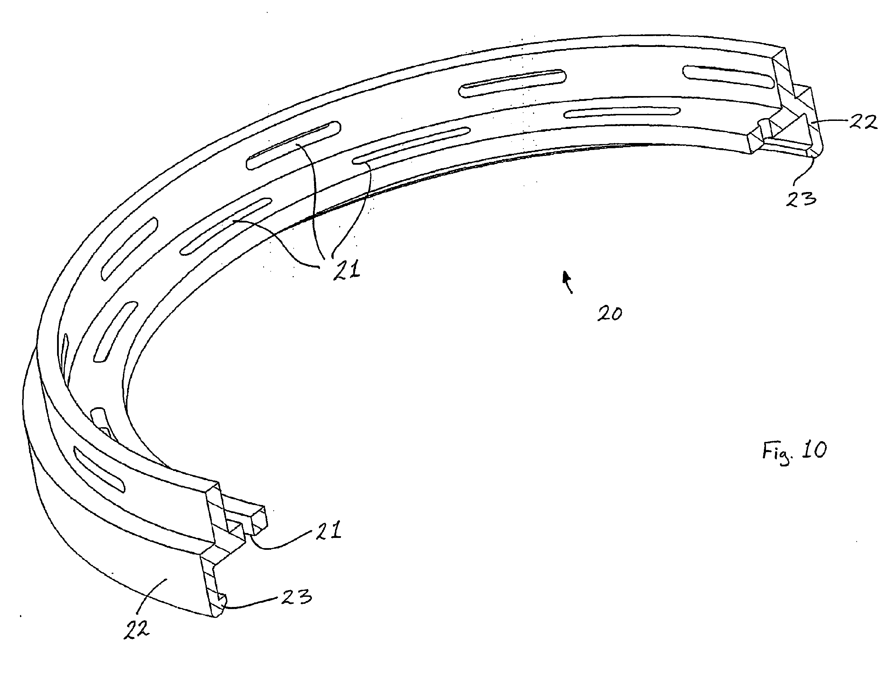

Figs. 9 and 10 illustrate a mounting ring 20 suitable for use when mounting the device 1 of Figs. 1 to 8 to a wound retractor device. A wound retractor device is typically employed to retract the sides of a wound opening to the abdomen 4. The ring 20 is sufficiently rigid to enable the device 1 to be mounted to the retractor device in a stable manner.

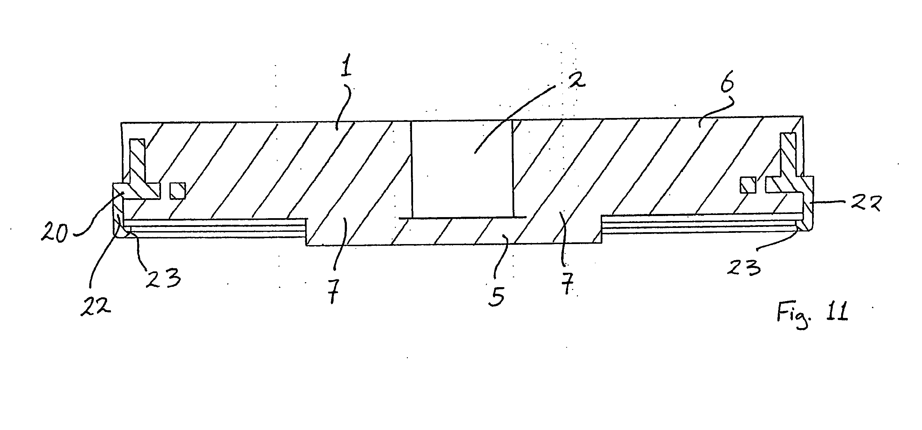

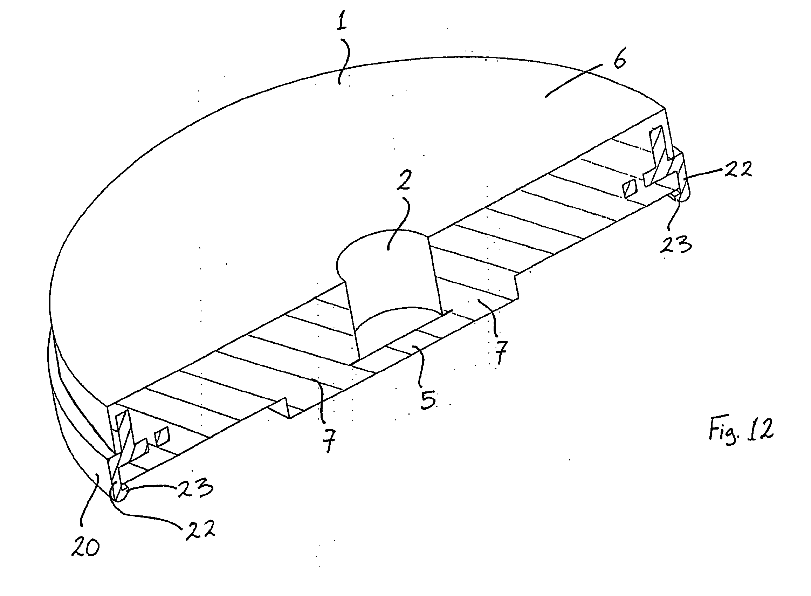

The ring 20 includes a series of oblong openings or slots 21 therein, in both the vertical and horizontal planes. The slots 21 assist in obtaining a secure attachment of the device 1 to the ring 20 (Figs. 11 and 12).

One method of attaching the device 1 to the ring 20 is by overmoulding a part of the second sealing member 6 around parts of the ring 20. The slots 21 thus provide pathways for the second sealing member 6 to mould around parts of the ring 20 to fixedly attach the device 1 to the ring 20.

Because the first and second sealing members 5, 6 are moulded integrally, the process of moulding the device 1 and attaching the device 1 to the ring 20 may be performed in a single moulding step.

It will be appreciated that the device 1 may be fixedly attached to the mounting ring 20 in a variety of possible alternative ways. For example, the device 1 may be securely attached to the ring 20 using an adhesive or any other suitable fixing means.

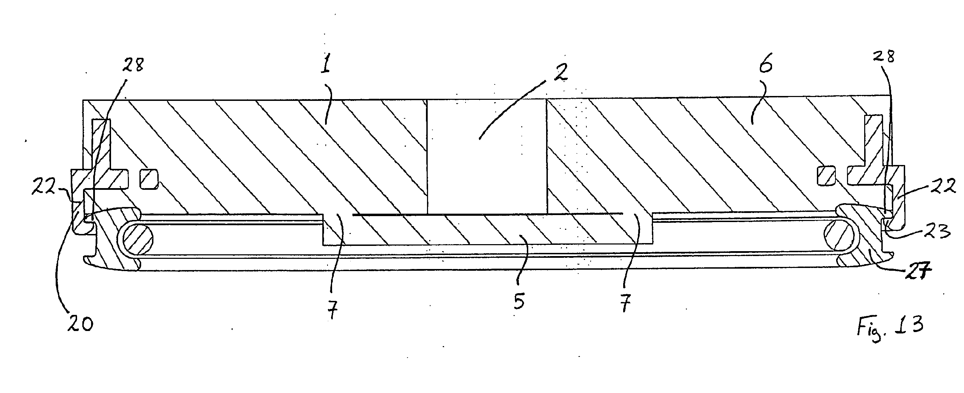

The ring 20 includes a downwardly extending rim 22 having an inwardly protruding engagement lip 23. The lip 23 is particularly suitable for mounting the ring 20 and the overmoulded device 1 to a retractor device in a snap-fit manner. For example, in the case of the wound retractor device 27 if Fig. 13, the retractor device 27 has a co-operating outwardly extending ledge 28 for snap-fit mounting of the ring 20 and overmoulded device 1 to the wound retractor device 27.

As illustrated in Fig. 13, the second sealing member 6 protrudes distally to engage the wound retractor device 27, when the ring 20 and overmoulded device 1 are mounted to the retractor device 27. This arrangement creates a gas-tight seal between the device 1 and the wound retractor device 27, and thus acts as a further means of preventing loss of insufflation gases.

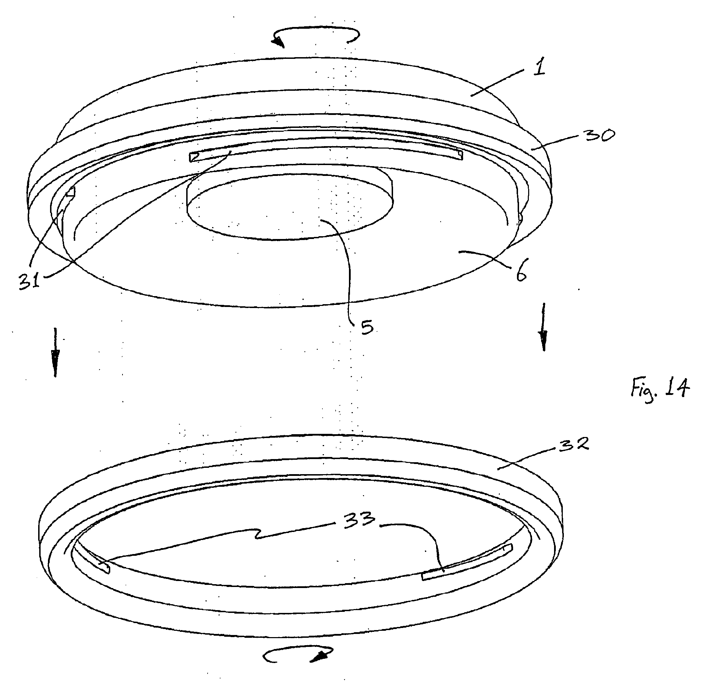

The mounting ring may be mounted to a wound retractor device in any suitable manner. For example, screw-thread formations 31 may be provided on the ring 30, and co-operating screw-thread formations 33 may be provided on the wound protector device 32, as illustrated in Fig. 14, for screw-thread mounting of the ring 30 and overmoulded device 1 to the retractor device 32.

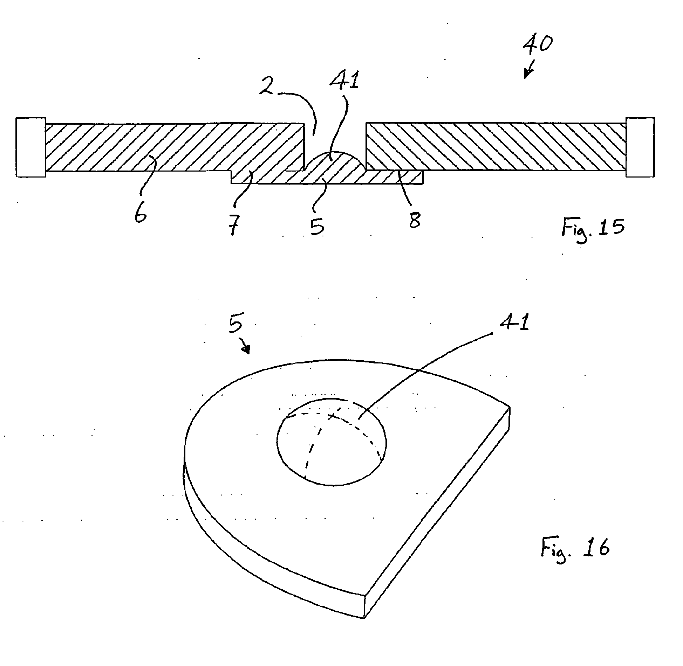

In Figs. 15 and 16, there is illustrated another surgical sealing device 40, which is similar to the device 1 of Figs. 1 to 8, and similar elements in Figs. 15 and 16 are assigned the same reference numerals.

In this case, the first sealing member 5 includes a hemispherical portion 41. In the closed configuration, the hemispherical portion 41 extends partially into the passageway 2 to achieve a particularly effective seal.

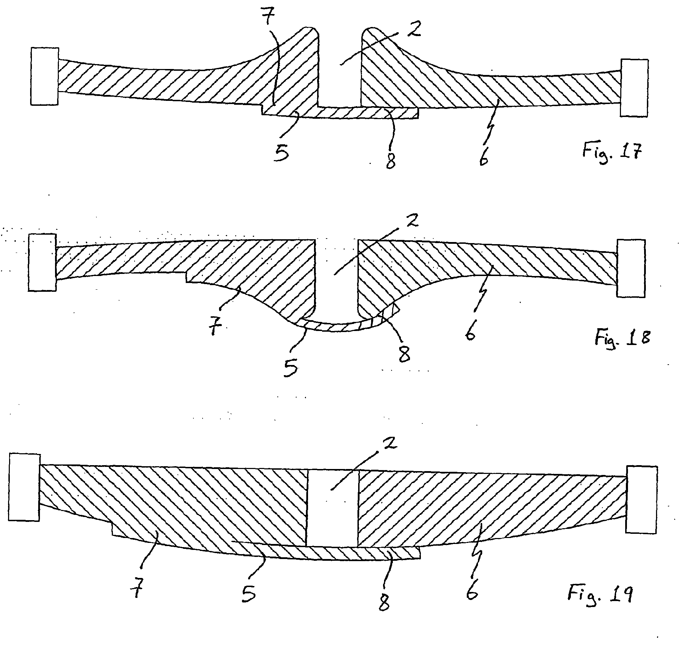

To ensure that the first sealing member 5 engages the second sealing member 6 around the entire periphery of the distal end of the passageway 2 in the closed configuration, the thickness of the second sealing member 6 may vary across the width of the second sealing member 6. A variety of possible configurations for the second sealing member 6 are illustrated in Figs. 17 to 19. By arranging for the thickness of the second sealing member 6 to be at a maximum adjacent the passageway 2, the sealing effect may be enhanced.

By arranging for the proximal side of the second sealing member 6 to curve proximally away from the distal end of the passageway 2 (Figs. 18 and 19), it has been found to result in a better sealing arrangement in certain clinical applications. The first sealing member 5 is also curved in these cases to mate with the curved second sealing member 6 in the closed configuration (Figs. 18 and 19).

In the embodiments of Figs. 17 and 18, the raised material of the second sealing member 6 puts weight on the overlapping first sealing member 5 to maintain an effective seal. The curved/domed surfaces of the first sealing member 5 and the second sealing member 6 improve the sealing in Fig. 19.

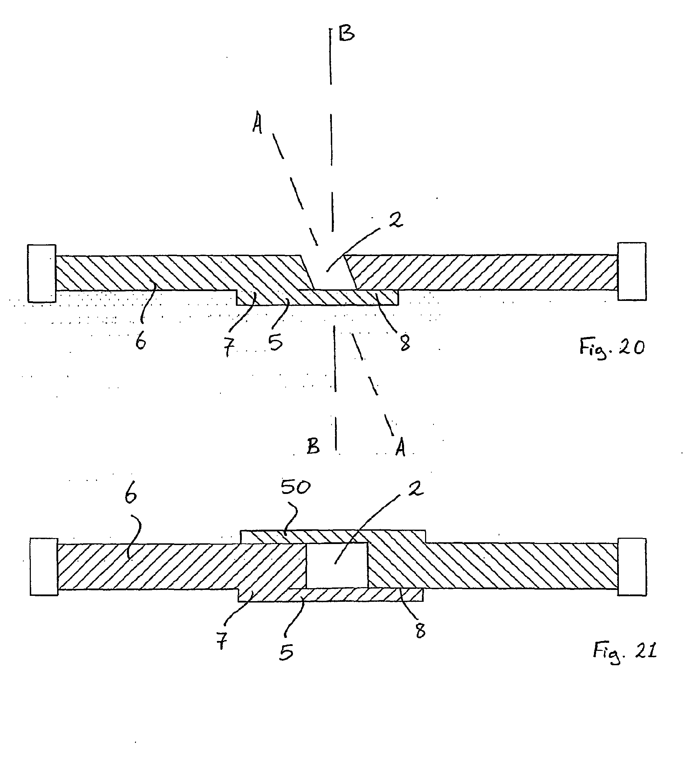

The passageway 2 may be arranged so that the passageway longitudinal axis A-A subtends an acute angle with the longitudinal axis B-B of the wound opening, and the passageway 2 is angled towards the detachment region 8, as illustrated in Fig. 20.

In this manner, the passageway 2 effectively guides the instrument or surgeon's hand in the direction of the detachment region 8 of the first sealing member 5. This assists in a faster and smoother insertion through the passageway 2 and retraction of the first sealing member 5.

An additional sealing member 50 may be provided integrally moulded with the second sealing member 6, as illustrated in Fig. 21. This fourth sealing member 50 is similar to the first sealing member 5, and is also provided in the form of an overlapping flap. In the closed configuration, the fourth sealing member 50 overlaps the entire periphery of the proximal opening of the passageway 2. In this manner, the overlap of the fourth sealing member 50 over the second sealing member 6 creates a third sealing valve to achieve a gas-tight seal across the wound opening. The fourth sealing member 50 is movable relative to the second sealing member 5 from the closed configuration to the open configuration to facilitate insertion of an instrument or a surgeon's hand into the passageway 2.

In this case, the fourth sealing member 50 is of the same gelatinous elastomeric material as the first and second sealing members 5, 6, and may be integrally formed.

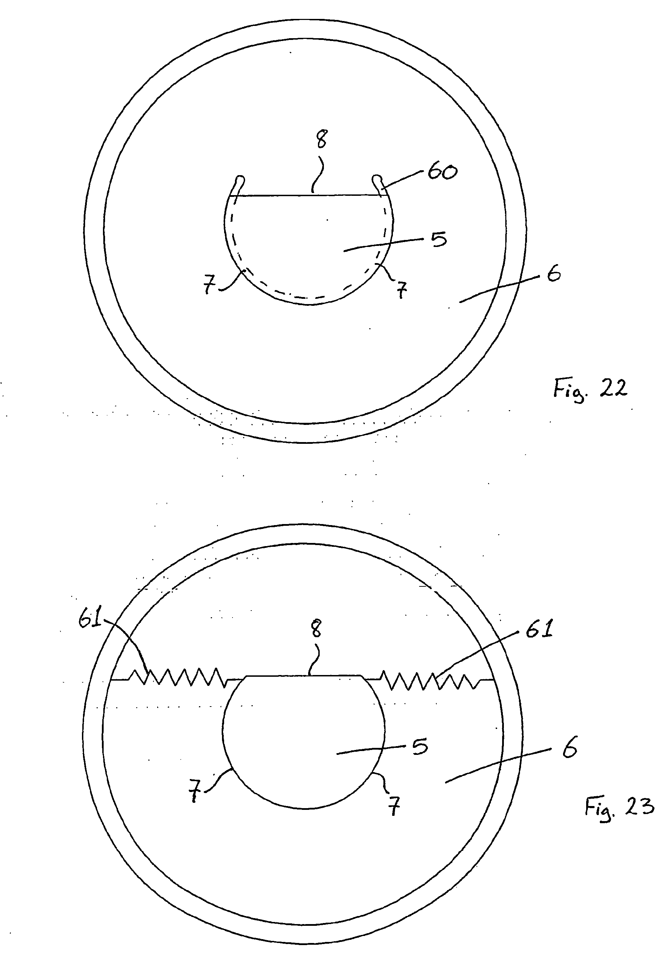

A biasing member may be provided to bias the first sealing member 5 towards the closed configuration. For example, Fig. 22 illustrates a "U"-shaped resilient leaf-spring member 60 for tensioning the detachment region 8 of the first sealing member 5 in the plane of the first sealing member 5. By tensioning in this plane, the leaf-spring 60 biases the detachment region 8 of the first sealing member 5 into engagement with the second sealing member 6. In this manner, an effective seal may be achieved.

Fig. 23 illustrates an alternative resilient biasing member. In this case two springs 61, 62 are provided extending between the detachment region 8 of the first sealing member 5 and the second sealing member 6 to tension the first sealing member 5 in the plane of the first sealing member 5.

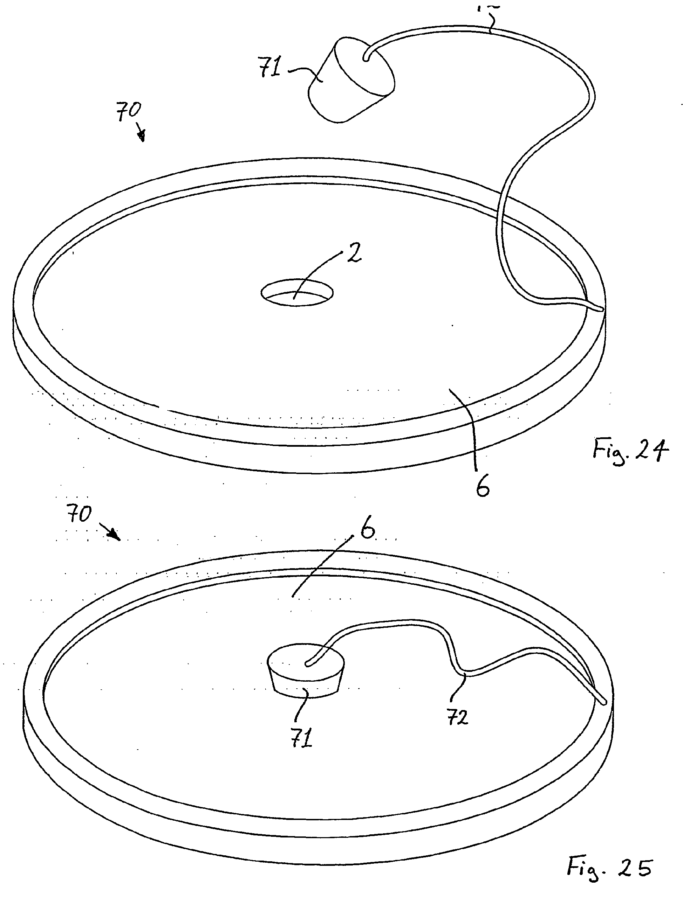

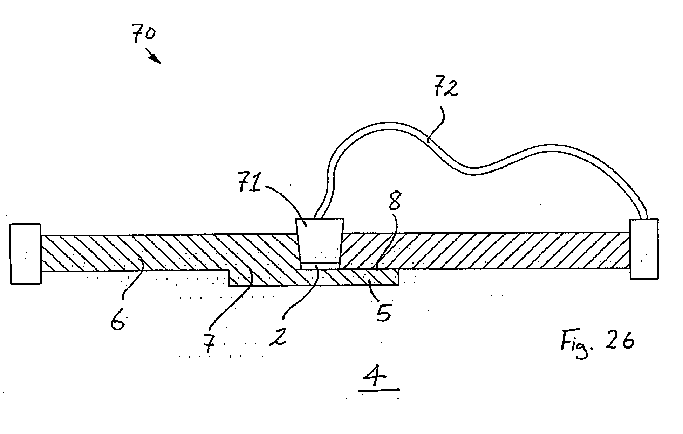

Figs. 24 to 26 illustrate another surgical sealing device 70, which is similar to the device 1 of Figs. 1 to 8, and similar elements in Figs. 24 to 26 are assigned the same reference numerals.

On this case, the device 70 comprises a plug 71 for insertion into the passageway 2 to close the passageway 2. By closing the passageway 2 with the plug 71, this acts as a further seal to prevent loss of insulation gases, for example during initial insufflation of the abdomen 4.

The plug 71 is attached by means of a cord to the second sealing member 6.

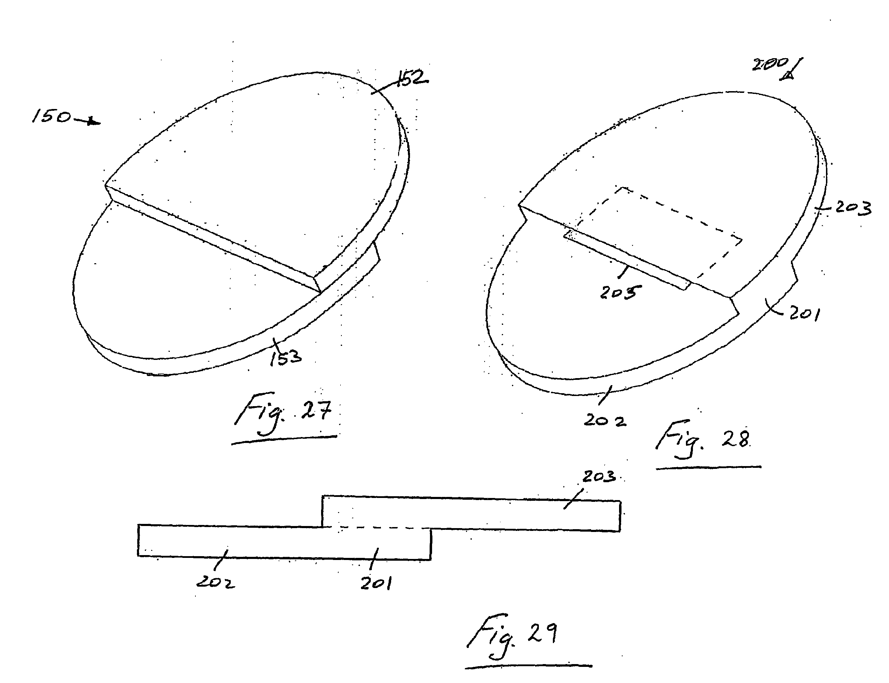

Referring now to Fig. 27 there is illustrated another surgical sealing device 150. In this case the device 150 has an overlap valve defined by a first sealing member 153 partially overlapping a second sealing member 152. The properties of the elastomeric material of the device 150, such as the sealing members 152, 153, is such as to closely surround an inserted object. Thus, there is substantially no leakage path between the inserted object and the valve body. Consequently, the device 150 may be used without the necessity for another valve, there being sufficient sealing engagement between the sealing members 152, 153 and the inserted object to substantially prevent insufflation gas leakage.

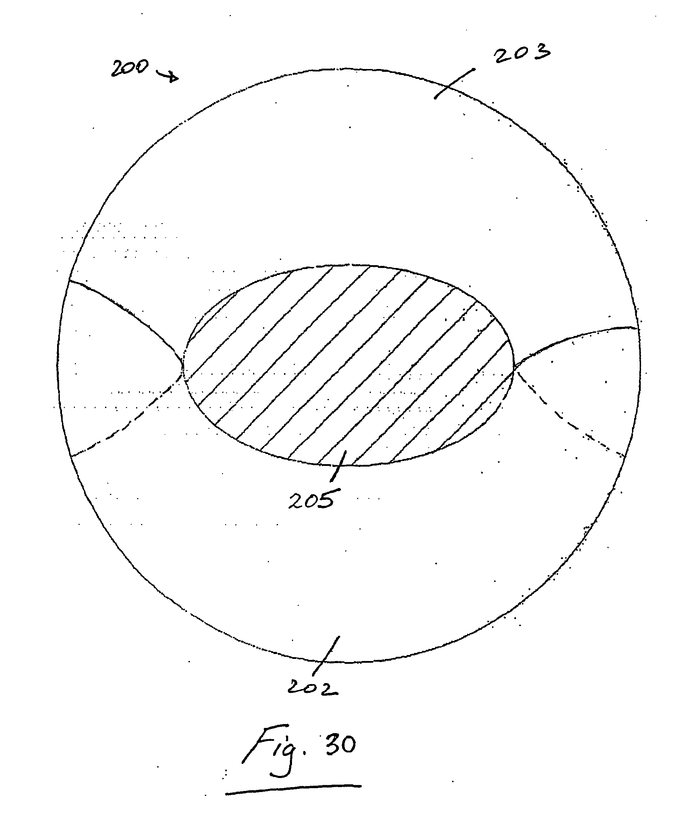

Referring to figs. 28 to 30, there is illustrated another surgical scaling device 200. The device 200 is formed from a highly elastomeric material, such as a silicone based gel. The first sealing member 202 and the second sealing member 203 have relatively thin outer portions. At the attachment region 201, the sealing members 202, 203 have a relatively thick central portion. The central portion has a detachment region slit 205 therein which extends obliquely with respect to the axis of an incision to provide enhanced scaling between the marginal edge of the accessway defined by the slit 205 and an object passing therethrough, as illustrated in Fig. 30. The valve in this case has high elasticity to comply with an object passing therethrough, and a secondary valve may therefore not be required.

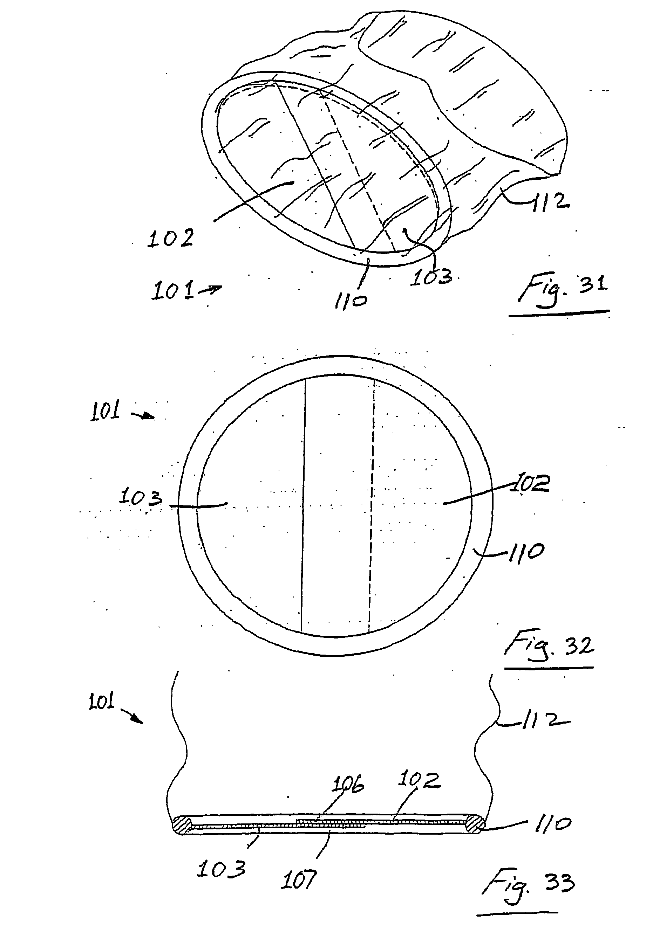

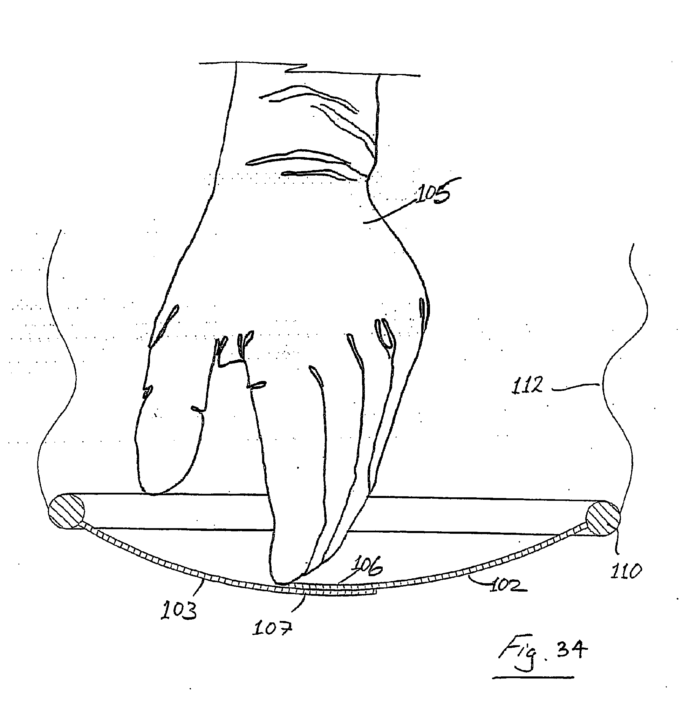

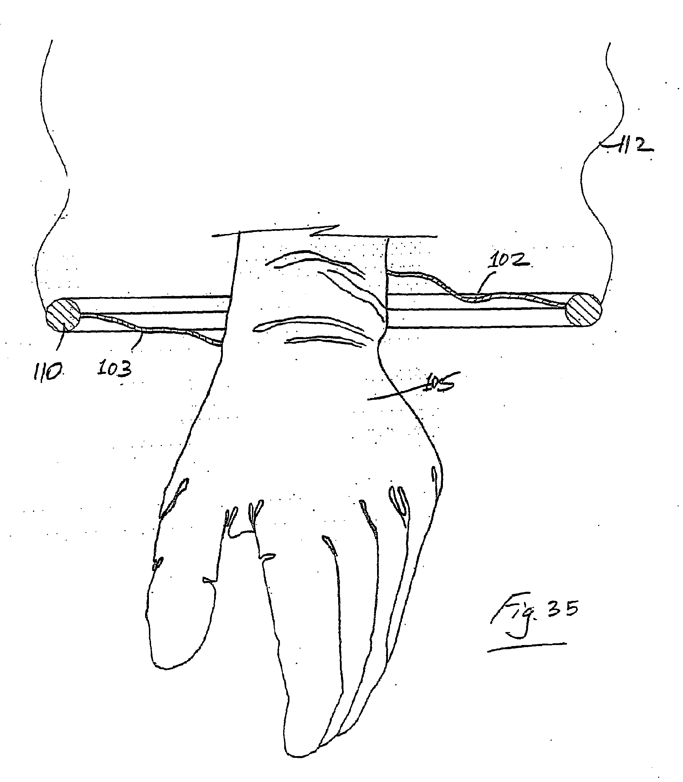

Referring to Figs. 31 to 35, there is illustrated a surgical sealing device 101 for use in a surgical procedure, such as laparoscopic surgery, or hand assisted laparoscopic surgery. The device 101 comprises a first scaling member 102 and a second sealing member 103 which have a normally closed configuration (Figs. 33 and 34) and an open configuration (Fig. 35). The sealing members 102, 103 extend across an opening, such as a retracted incision, and partially overlap each other in the closed configuration (Fig. 34). The sealing members 102, 103 are movable on insertion of an object, such a surgeon's hand/forearm 105, to facilitate access between the sealing members 102, 103 (Fig. 35).

The scaling members 102, 103 have respective overlapping portions 106, 107 which overlap with each other in the closed configuration. The sealing members 102, 103 are generally planar, with the planes of the overlapping portions 106, 107 being slightly offset. The overlapping portions 106, 107 are at least partially engaged with one another in the closed configuration. In this case, the sealing members 102, 103 are of a resilient and elastomeric sheet material such as rubber, silicone or latex. The engagement force between the overlapping portions 106, 107 is overcome by the insertion of an object, such as a surgeon's hand.

In this case the sealing members 102, 103 are each mounted to a mounting ring in the form of an O-ring 110. A sleeve 112 extends proximally from the mounting O-ring 110.

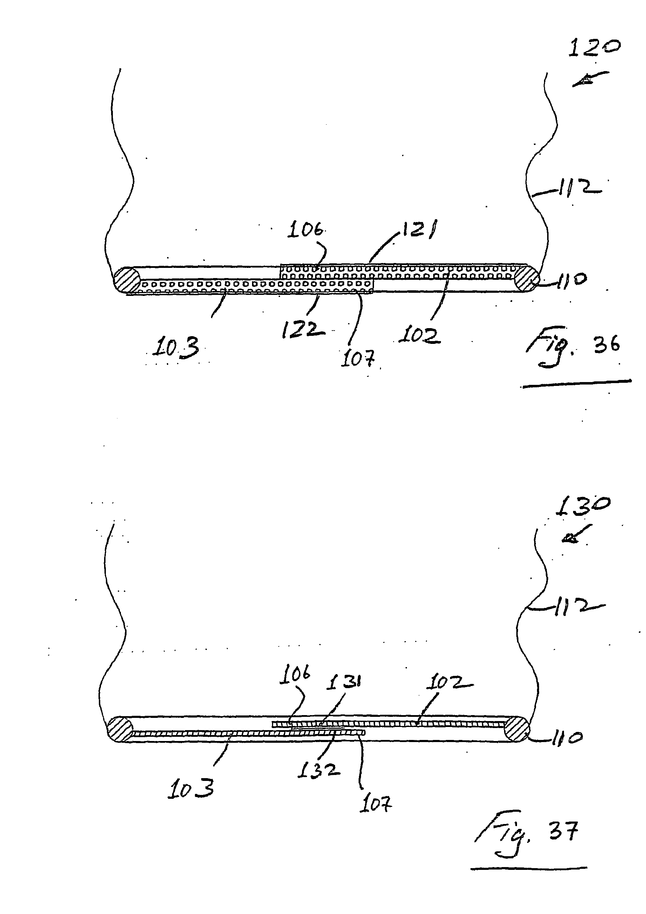

Referring to Fig. 36 there is illustrated another surgical sealing device 120, in which parts similar to the device 101 of Figs. 31 to 35 are identified by the same reference numerals.

The sealing members 102, 103 are, in this case, of an elastic material such as a gel, neoprene, rubber or silicone which may optionally have facings 121, 122 on the non-engaged side. Indeed, there may be a facing on the engaged side to enhance the engagement. In this case, the facings may have some adhesive or tackiness properties.

Referring to Fig. 37, in this case, a surgical sealing device 130 has magnets 131, 132 situated in the overlap portions 106, 107 respectively to enhance engagement between the sealing members 102, 103 to maintain the seal in the closed configuration. The co-operating magnetic elements 131, 132 act as biasing members to bias the device 130 towards the closed configuration.

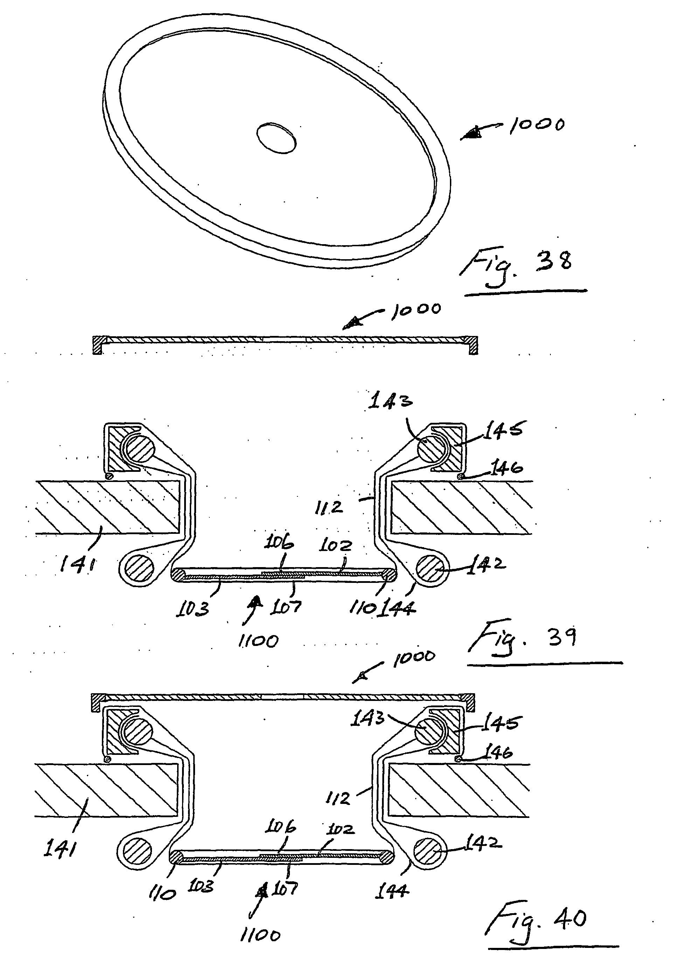

Referring to Fig. 38 there is illustrated a third sealing member 1000, in this case in the form of a lip seal type valve 1000 which may be used as a second sealing valve. The lip seal 1000 has a passageway extending therethrough, through which an object may be inserted. The passageway effects a seal between the sidewall of the passageway and the object inserted therethrough. The lip seal valve 1000 may be located distal to or within the first sealing valve, such as an overlap valve 1100. The overlap valve 1100 may be of any of the types described above, and like parts are assigned the same reference numerals.

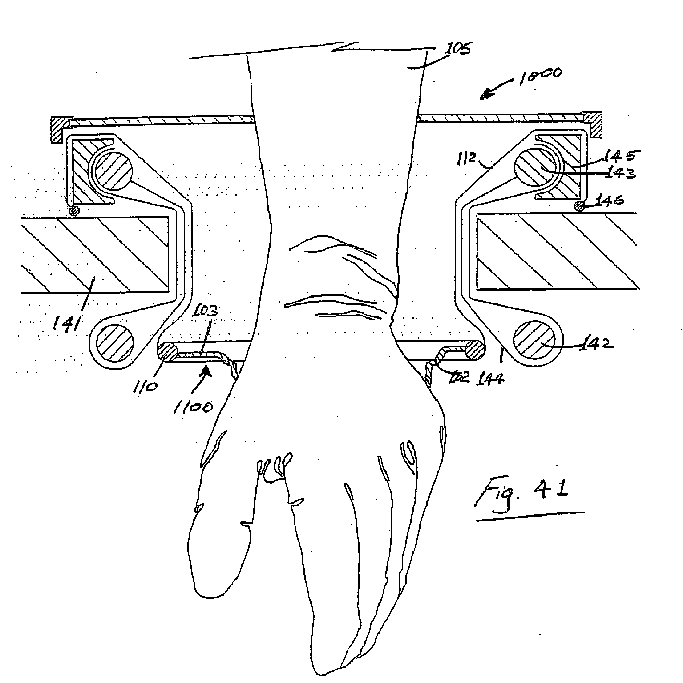

Referring to Figs. 39 to 41, the overlap valve 1100 is located just inside an incision. The valve 1100 is coupled to a retractor located in the incision at a surgical site, such as in an abdominal wall 141.

The retractor may be of any suitable construction such as the retractors described in our US patent application published under No. 2001/0037053A, and/or US 6,582,364, and/or US 2005/0090717 A.

In this case, the retractor has a distal ring 142 and a proximal ring 143 with a retracting sleeve 144 extending therebetween, as illustrated. The retractor also has a proximal guide or mounting ring 145. In this case, the proximal ring 143 is located in a recess in the guide-mounting ring 145, however various other constructions are possible. The valve sleeve 112 is mounted to the retractor, in this case to the mounting ring 145. The distal end of the valve sleeve 112 has a reinforcing mounting ring 146 to facilitate coupling, as shown. In particular the reinforcing ring 146 may be hooked around the mounting ring 145 to mount the device 1100 to the retractor.

The lip seal 1000 is attached to the mounting ring 45 of the retractor.

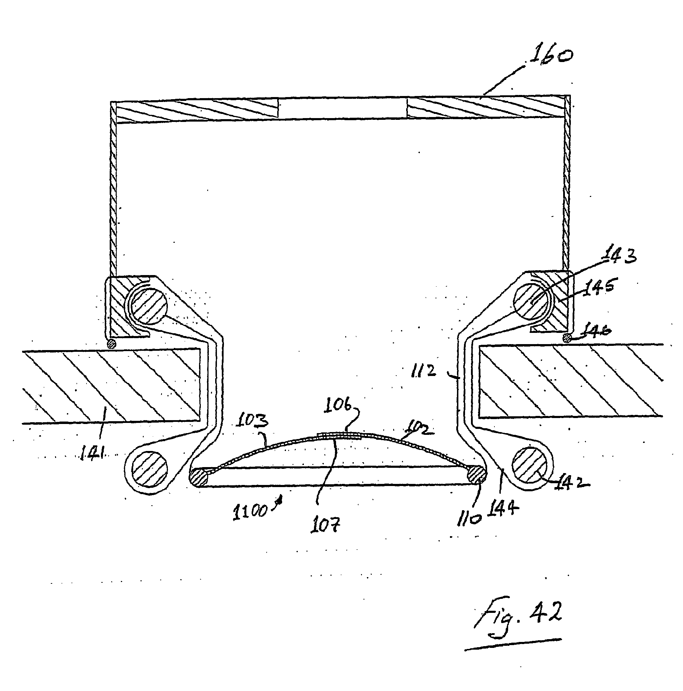

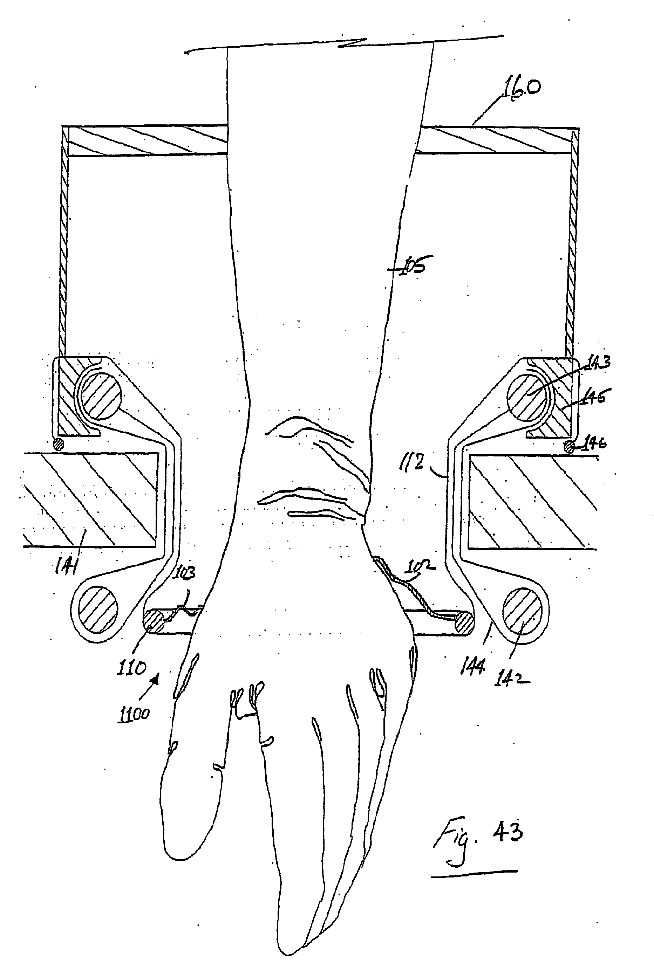

Referring to Figs. 42 and 43, there is illustrated another surgical assembly similar to that of Figs. 39 to 41, and like parts are identified by the same reference numerals. In this case, the second sealing valve is provided by a lip seal valve 160. The lip seal 160 may be of any suitable material such as silicone, gel or rubber.

The first sealing overlap valve 1100 is located within the abdomen distally of the wound opening, and the second sealing lip seal valve 160 is located externally of the abdomen proximally of the wound opening. As illustrated, the first sealing valve 1100 is longitudinally spaced apart from the second sealing valve 160.

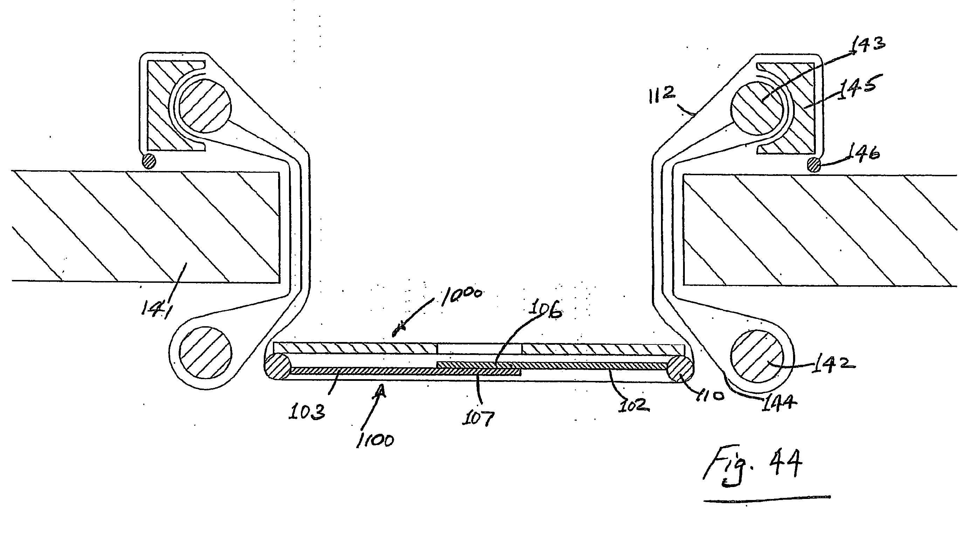

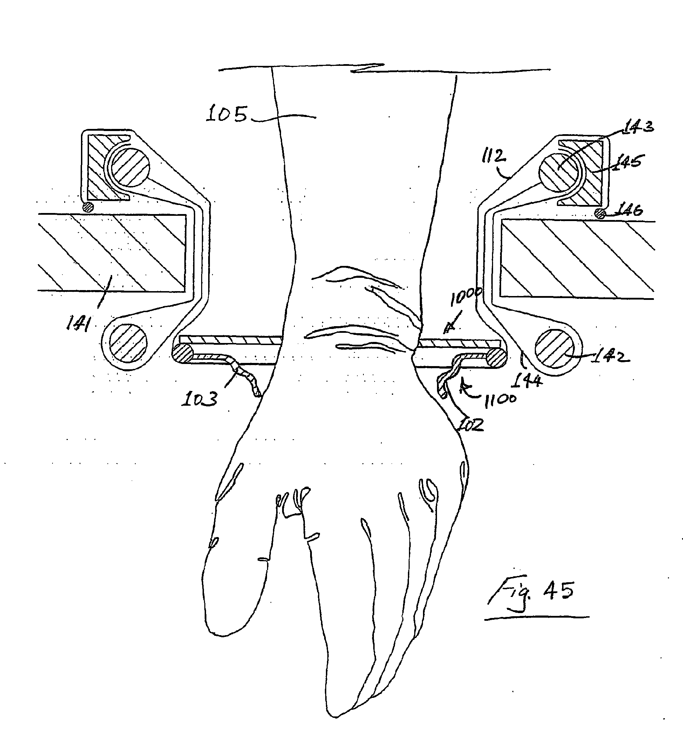

Referring to Figs. 44 and 45, there is illustrated another surgical assembly, which is similar to the assembly of Figs. 39 to 41, and similar elements in Figs. 44 and 45 are assigned the same reference numerals.

In this case, the second sealing valve lip seal 1000 is located just proximal of, adjacent and in close proximity to the first sealing overlap valve 1100. The lip seal 1000 may be attached to the mounting ring 110 of the overlap valve 1100. One advantage of this arrangement is the very low profile of the device. Both the first sealing valve 1100 and the second sealing valve 1000 are located, in use, within the abdomen distally of the wound opening, and adjacent to the distal end of the wound retractor device.

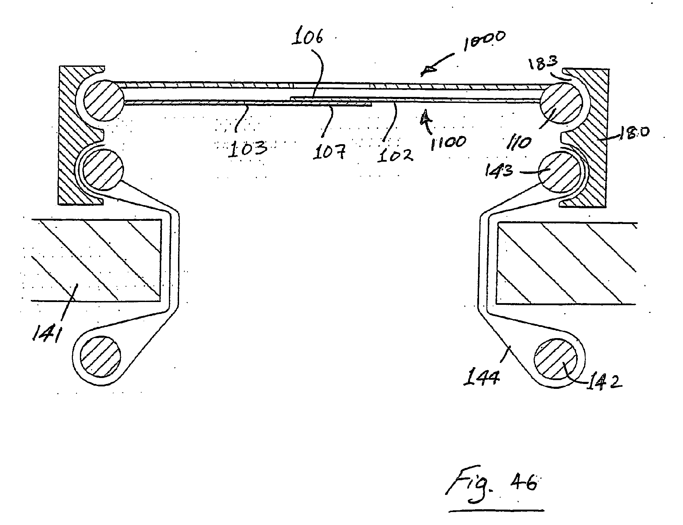

Referring to Fig. 46, in this case the retractor has an outer mounting ring 180 of the type described above with reference to Figs. 39 to 41. The second sealing lip seal valve 1000 is attached to the mounting ring 110 of the first sealing overlap valve 1100 which in turn is located in an outer recess 183 of the mounting ring 180.

The first sealing valve 1100 is located adjacent and in close proximity to the second sealing valve 1000. Both the first sealing valve 1100 and the second sealing valve 1000 are located, in use, externally of the abdomen proximally of the wound opening, and adjacent to the proximal end of the wound retractor device.

As an alternative, one or both of the sealing valves may be located within the wound opening.

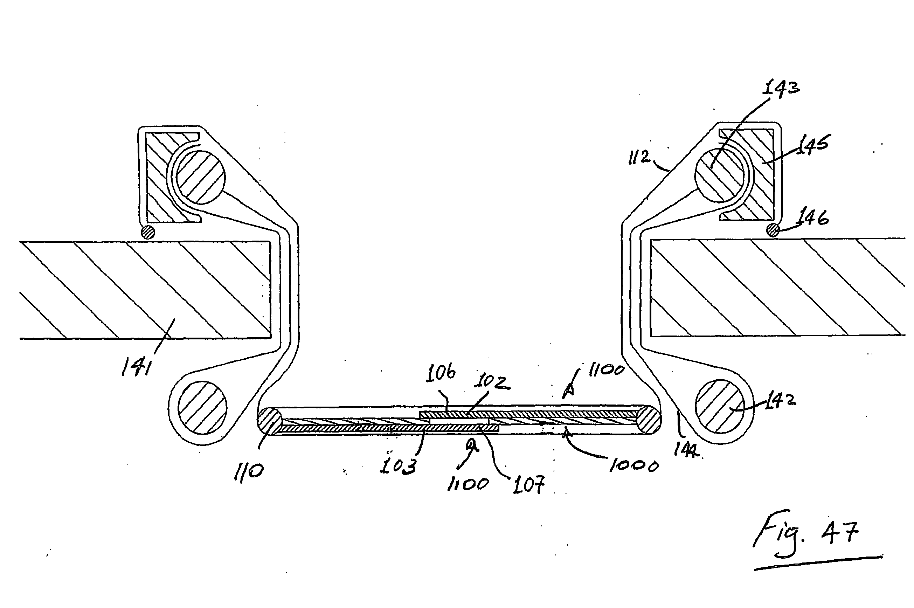

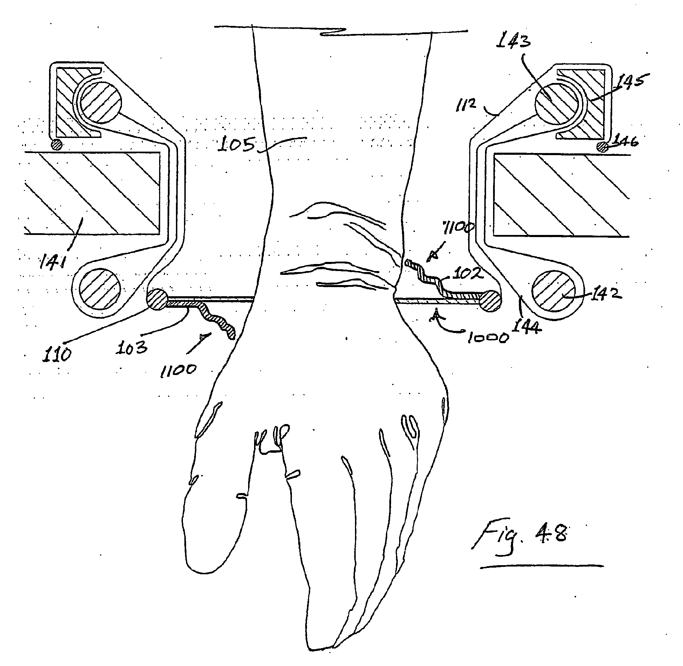

In Figs. 47 and 48, the second sealing valve lip seal 1000 is located within the first sealing overlap valve 1100. The body of the lip seal 1000 is located between the flaps 102, 103 of the overlap valve 1100. This device has a particularly low profile.

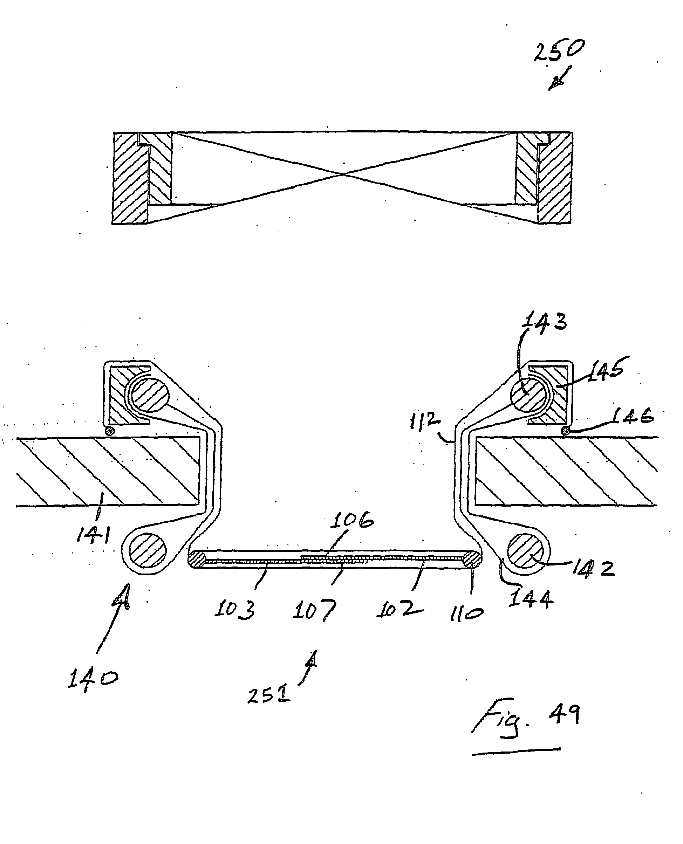

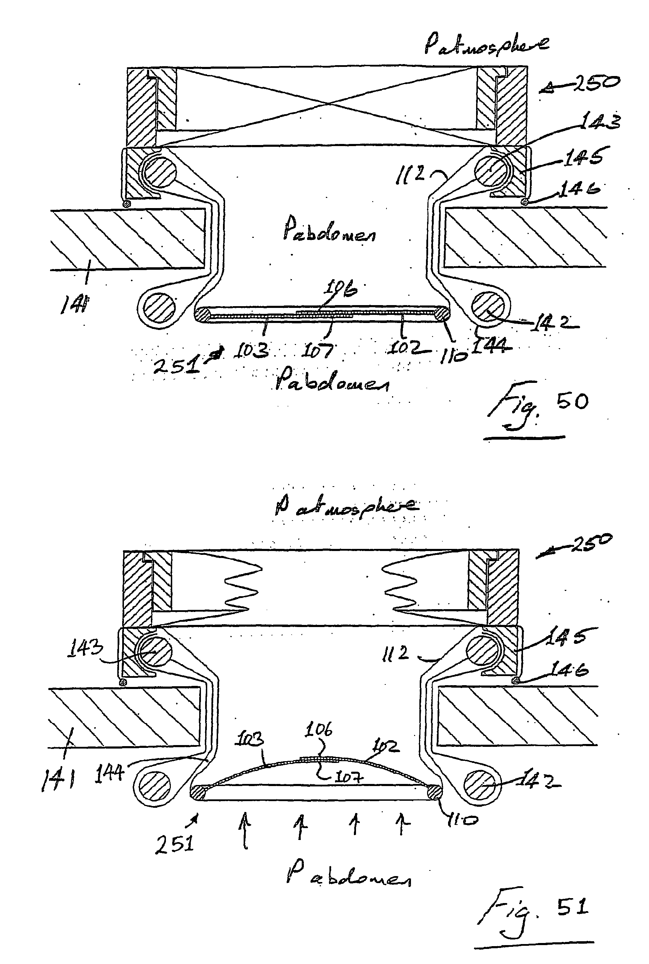

Referring to Fig. 49 to 54, there is illustrated another surgical sealing device 251 comprising a first sealing overlap valve of the type described above.

The mounting ring 145 provides a platform for mounting a second sealing valve 250, such as an iris-type valve to the retractor 140. In this case, the valve 250 may be regarded as a primary valve and the overlap valve as a secondary valve.

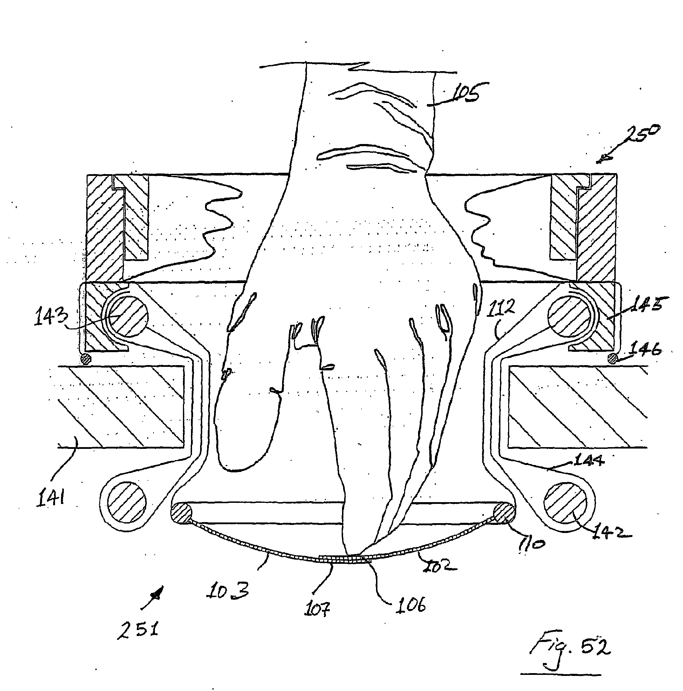

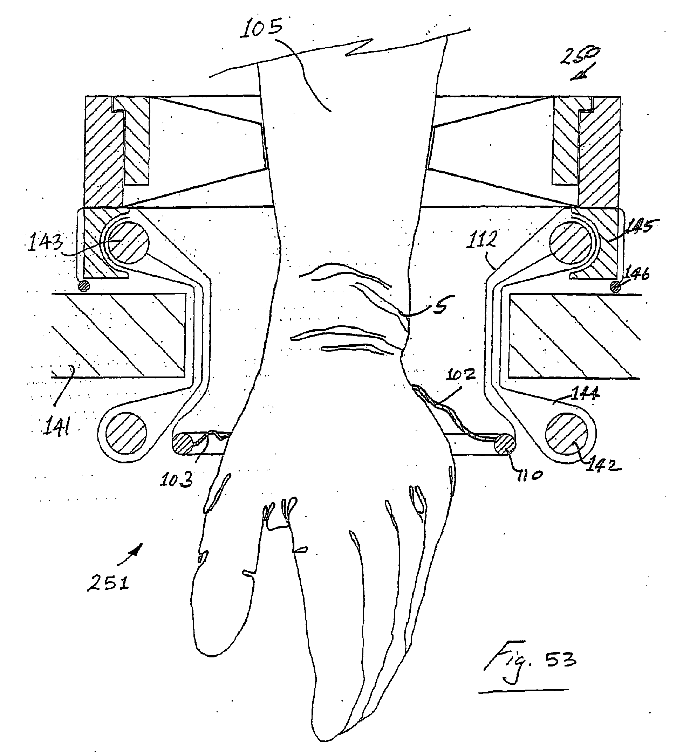

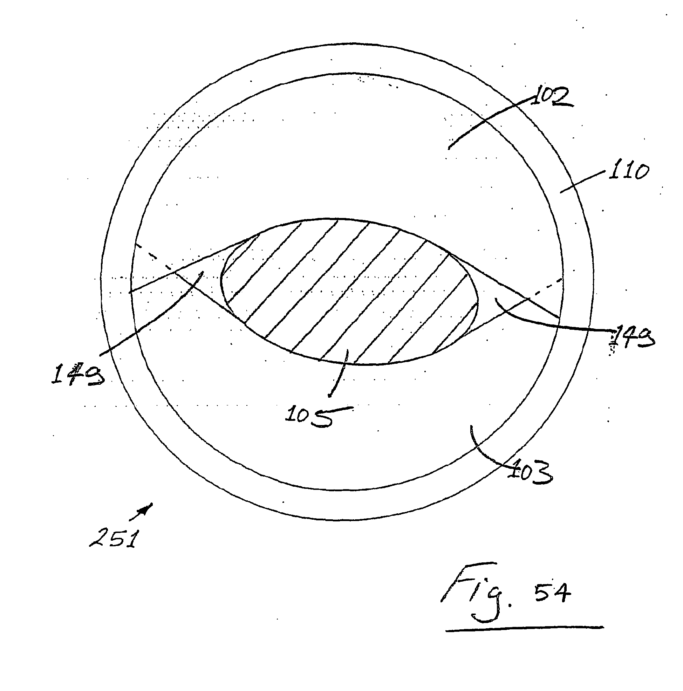

In use, the space beneath the abdominal wall 141 is insufflated. The primary valve 250 is normally closed and is opened for insertion of an object, such as a laparoscopic instrument or a surgeon's hand/forearm 105. On opening of the valve 250 the secondary overlap valve holds back the pressure of the gas inside the abdomen (Fig. 52). When the valve 250 has engaged around the surgeon's forearm 105 (Fig. 53), the primary valve 250 provides a seal preventing gas leakage between the surgeon's forearm 105 and the valve 250. The surgeon can then readily open the secondary seal of the overlap valve whilst maintaining pneumoperitoneum. Thus, sealed access is provided without substantial gas leakage from the abdominal cavity. It will be noted, especially from Fig. 54 that the secondary overlap valve, in this case, does not tightly seal around the surgeon's arm 105 but rather there are leak paths 149 between the arm 105 and the sealing members 102, 103. Gas leakage is held back by the sealed primary valve 250.

The valve system of the invention is easy to use by a surgeon and yet avoids substantial gas leakage. Because the valve 250 is sealed to a surgeon's arm, the secondary overlap valve can be readily and safely opened to allow access into the abdominal cavity. The secondary overlap valve provides an effective seal against gas leakage while a surgeon seals his arm to the primary valve 250. Upon opening of the secondary overlap valve, the primary valve 250 then provides the primary seal against gas leakage.

In this case, the sealing members 102, 103 are of a gel material. However it will be appreciated that the sealing members 102, 103 may alternatively be provided in the form of any suitable elastic material, for example neoprene.

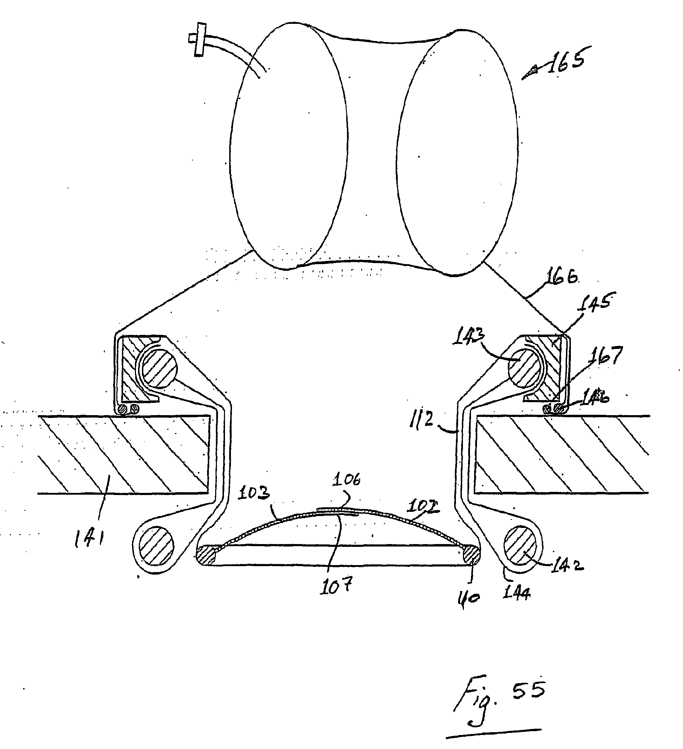

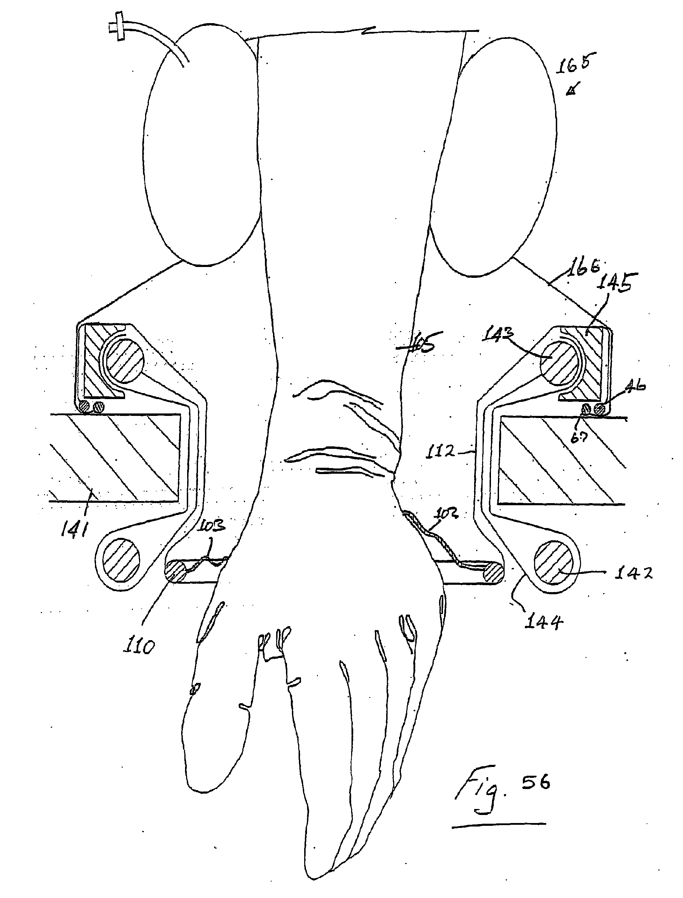

Referring to Figs. 55 and 56, there is illustrated another surgical assembly similar to that of Figs. 49 to 54. In this case the second sealing valve is an inflatable valve 165, which may be of the type described in our US 6,578,577, the whole contents of which are incorporated herein by reference. The valve 165 is in this case mounted to the retractor base by a mounting sleeve 166 having a reinforcing ring 167 which may be hooked over the mounting ring 145. Other mounting arrangements are possible. This type of inflatable valve/seal provides for easy and rapid hand insertion and withdrawal.

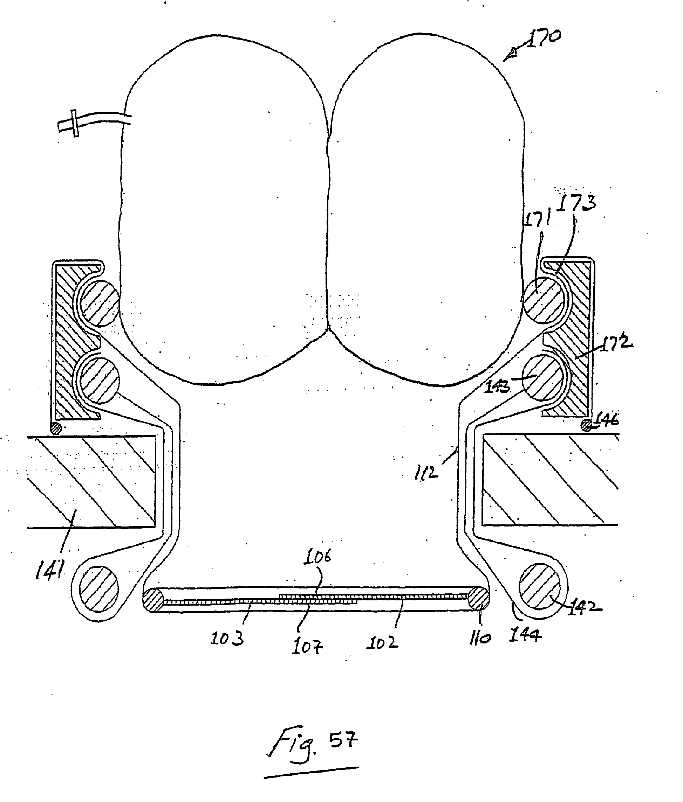

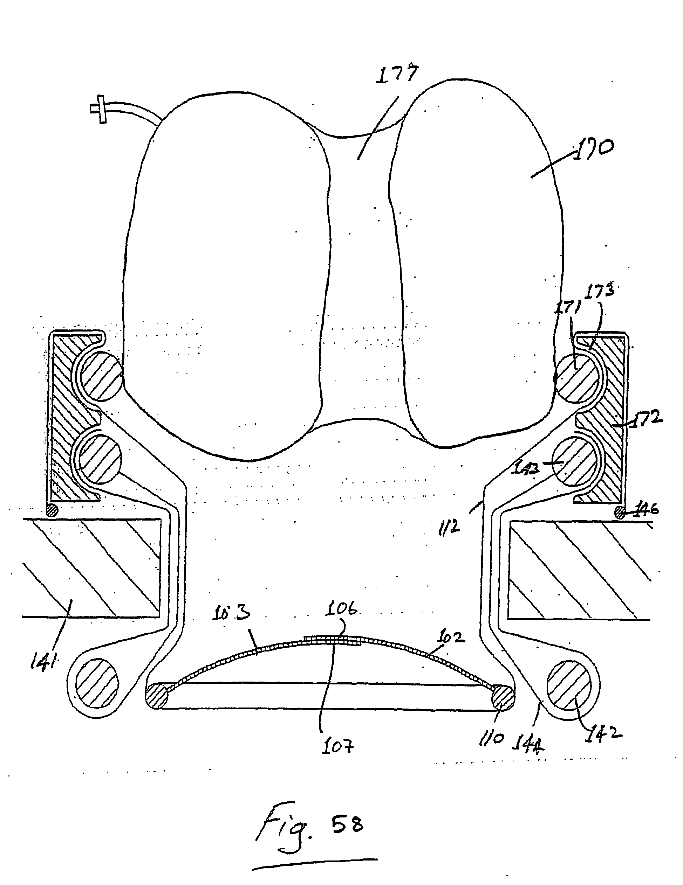

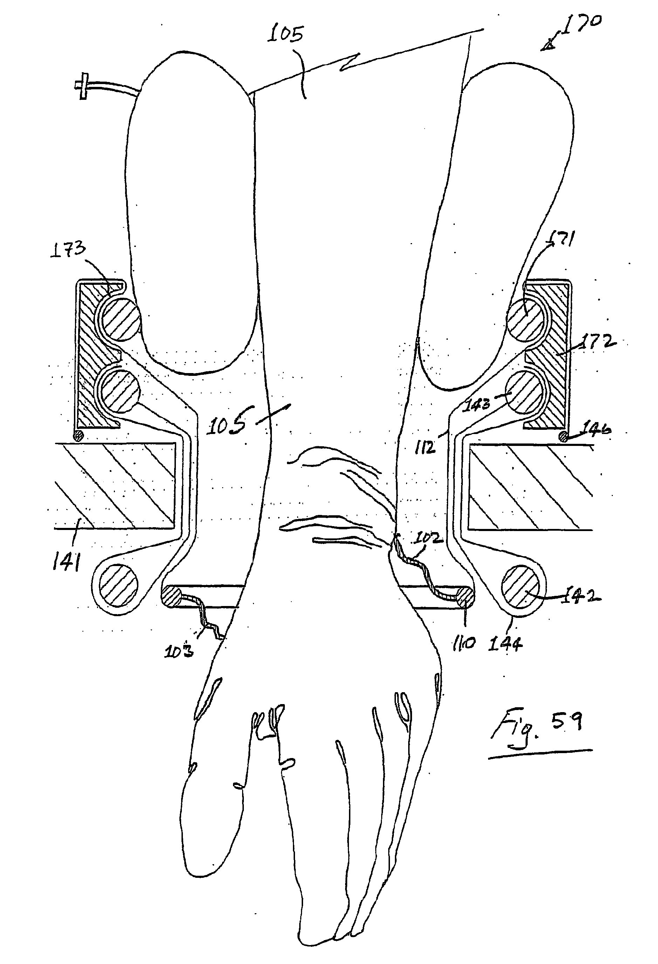

Referring to Figs. 57 to 59, there is illustrated another surgical assembly which is similar to that of Figs. 55 and 56. In this case the second scaling valve 170 is inflatable. The valve 170 has an attachment ring 171 for attachment to a mounting ring 172 of the retractor. In this case the attachment ring 172 has a recess 173 to accommodate the attachment ring 171 of the valve 170. In Fig. 57, an access channel 177 through the valve 170 is illustrated in a closed scaling configuration. In Figs. 58 and 59, the access channel 177 has been opened to sealingly engage a surgeon's arm 105 extending therethrough.

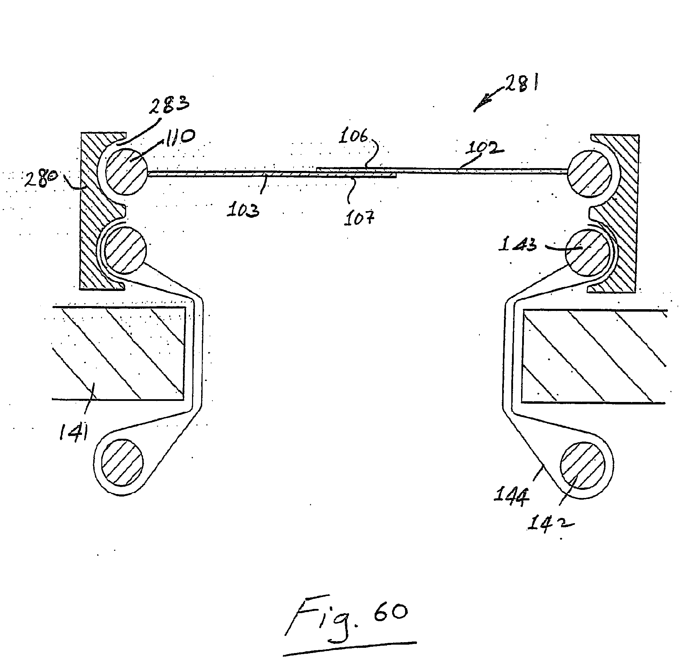

In some of the described embodiments above the first sealing overlap valve is located adjacent to a distal end of the incision. It is also possible to provide the first sealing overlap valve externally of the abdomen proximally of the wound opening. For example, as illustrated in Fig. 60, a retractor may have a mounting ring 280, and the mounting ring 110 of the first scaling overlap valve 281 may be mounted to the mounting ring 280, for example by engagement in a recess 283 in the mounting ring 280, as illustrated. In this case, a valve sleeve is not necessary.

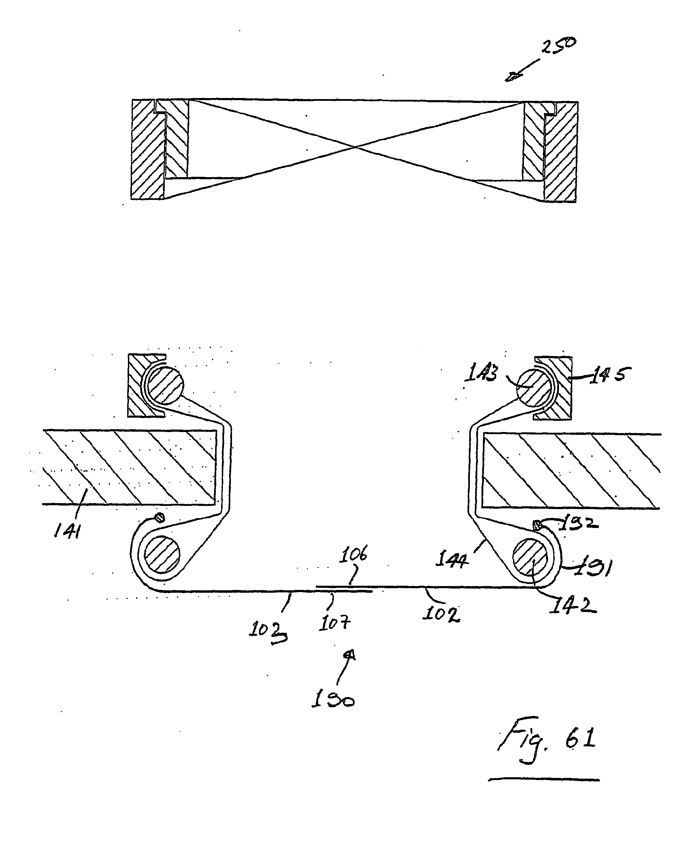

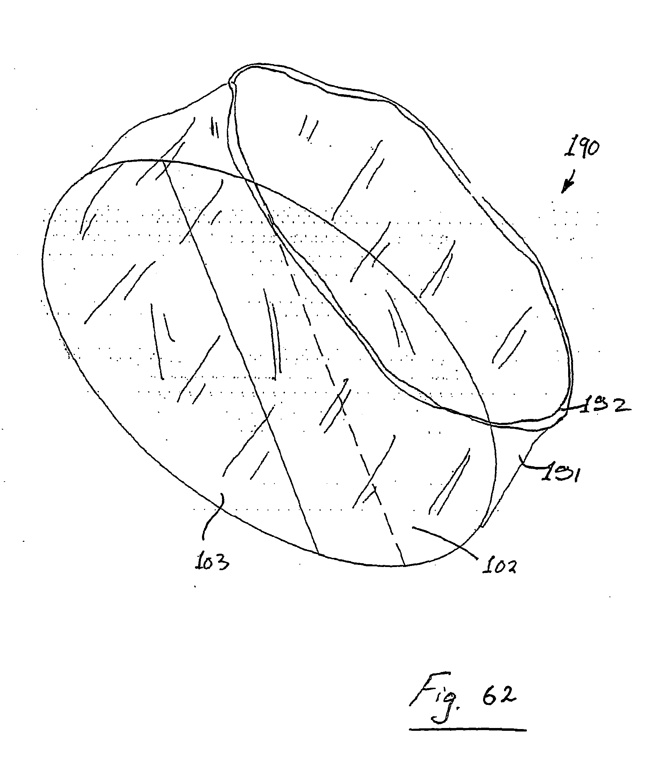

Various other mounting arrangements are possible. For example, as illustrated in Figs. 61 and 62, the first sealing overlap valve 190 may be mounted by hooking over the distal ring 142 of the retractor. In this case, the valve 190 has a cuff 191 with a reinforcing ring 192 to facilitate ease of mounting to the retractor.

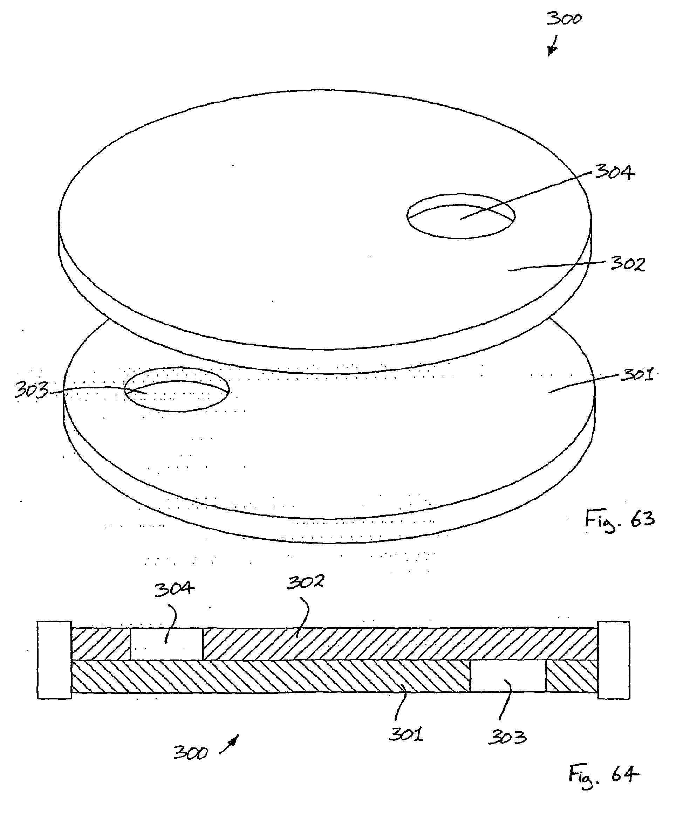

Referring to Figs. 63 and 64, there is illustrated a further surgical sealing device 300. The device 300 comprises a first scaling member 301 and a second sealing member 302. Each sealing member 301, 302 has a passageway 303, 304 extending therethrough. The passageways 303, 304 are arranged out of alignment with respect to one another. In this manner, the first sealing member 301 overlaps the entire periphery of the distal end opening of the passageway 304, and the second scaling member 302 overlaps the entire periphery of the proximal end opening of the passageway 303. Thus in the closed configuration illustrated in Fig. 64, the device 300 maintains an effective seal across a wound opening.

The sealing members 303, 304 are of an elastomeric gel material. The scaling members 303, 304 may therefore be manipulated to align the two passageways 303, 304, and thereby facilitate passage of an objet, such as a laparoscopic instrument or a surgeon's hand/forcarm, through the device 300 to access the abdomen.

Each passageway 303, 304 is in the form of a lip-seal, and is capable of effecting a gas-tight seal between the wall of the passageway 303, 304 and the object inserted therethrough. Thus insufflation gas is prevented from escaping from the abdomen while the object is inserted through the passageways 303, 304.

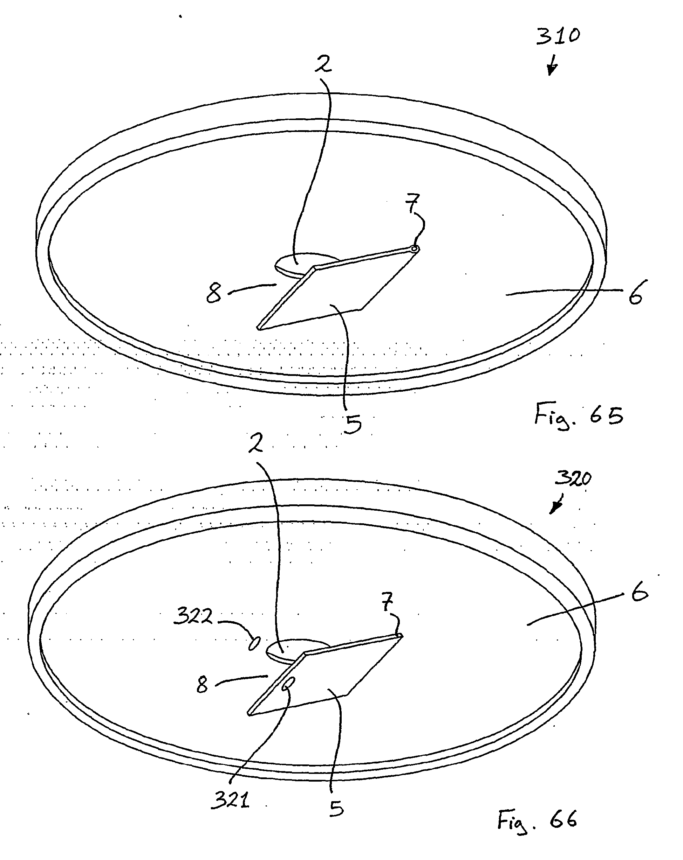

In Fig. 65, there is illustrated another surgical sealing device 310, which is similar to the device 1 of Figs. 1 to 8, and similar elements in Fig. 65 arc assigned the same reference numerals.

In this case, the first sealing member 5 is attached to the second sealing member 6 at the attachment region 7 by means of a hinge arrangement. The first sealing member 5 is thus movable in a hinging motion relative to the second sealing member 6 between the open and closed configurations.

For enhanced sealing in the closed configuration, a biasing member may he provided to bias the detachment region 8 of the first scaling member 5 towards engagement with the second sealing member 6, and thus bias the device 320 towards the closed configuration. A suitable biasing member is a magnetic element 321 on the first sealing member 5 and a co-operating magnetic element 322 on the second sealing member 6, as illustrated in Fig. 66.

Alternative biasing members include velero strips, or a latch.

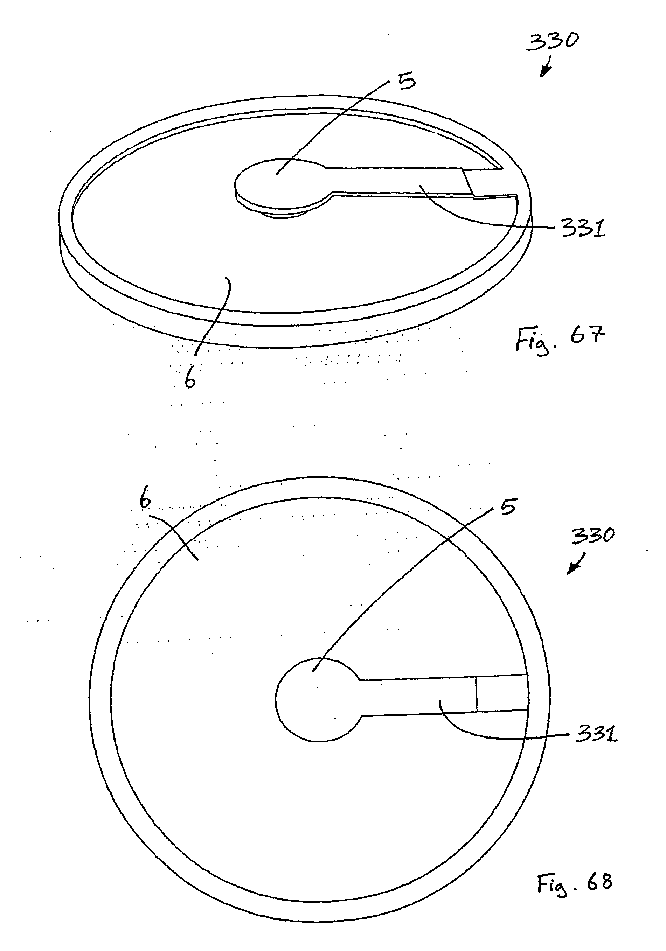

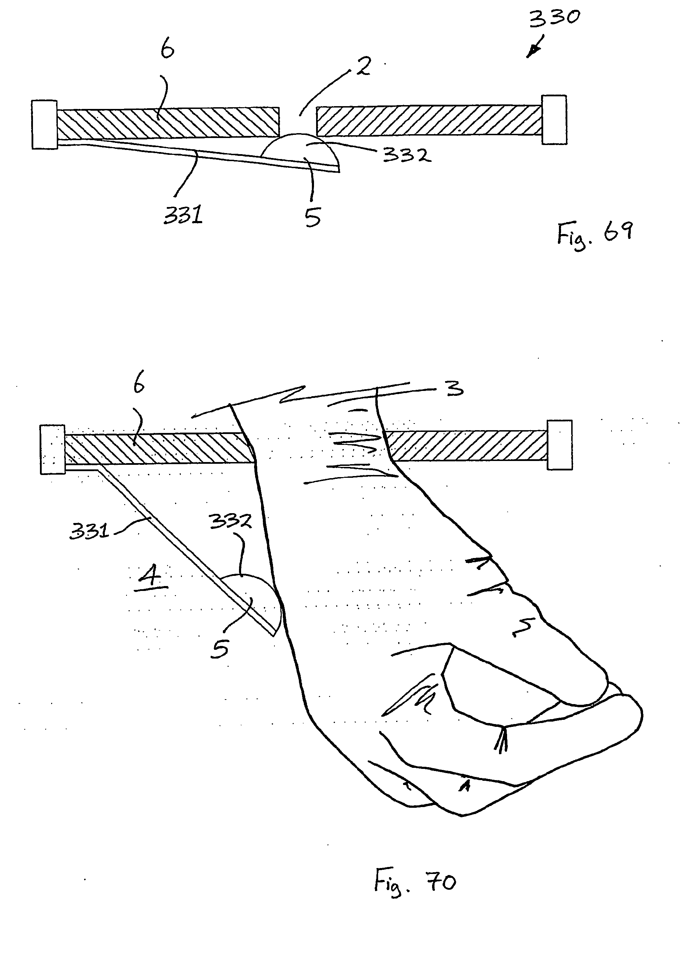

Figs. 67 to 70 illustrate another surgical sealing device 330, which is similar to the device 1 of Figs. 1 to 8, and similar elements in Figs. 67 to 70 are assigned the same reference numerals.

In this case, the first sealing member 5 is attached to the second sealing member 6 by means of a resilient cantilever arm 331. The arm 331 acts as a leaf-spring to bias the first scaling member 5 towards engagement with the distal end opening of the passageway 2, and thus bias the device 330 towards the closed configuration.

The first sealing member 5 has a partially spherical, curved portion 332 which partially extends into the passageway 2 in the closed configuration.

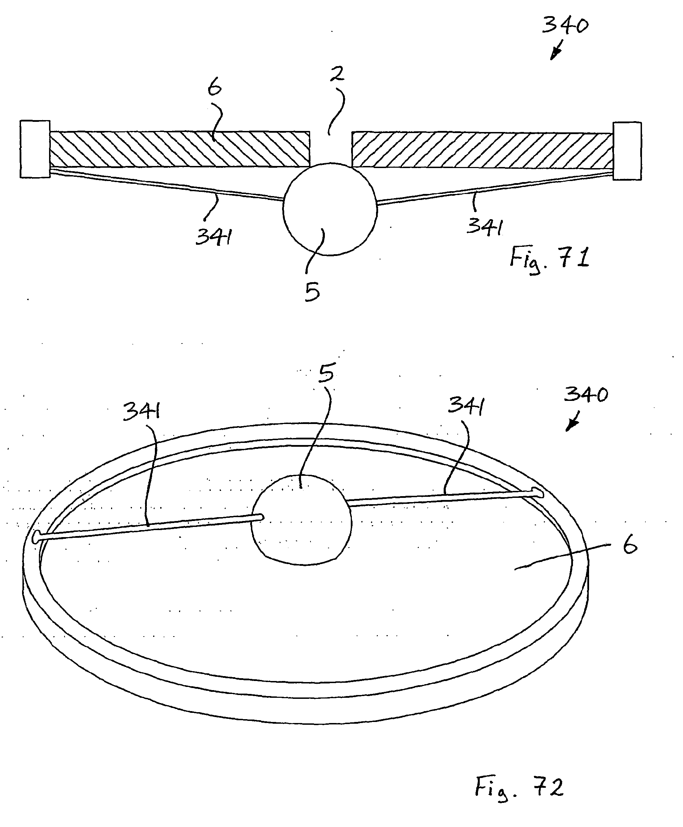

Referring to Figs. 71 and 72, there is illustrated another surgical sealing device 340, which is similar to the device 1 of Figs. 1 to 8, and similar elements in Figs. 71 and 72 are assigned the same reference numerals.

In this case the first sealing member 5 is provided in the form of a substantially spherical ball. In the closed configuration, the sphere 5 engages the distal end opening of the passageway 2 to effect a gas-tight seal. Part of the sphere 5 extends into the passageway 2 in the closed configuration.

The sphere 5 is attached to the distal side of the second sealing member 6 by means of two resilient cords 341. The cords 341 act as spring members to bias the sphere 5 towards engagement with the distal end opening of the passageway 2, and thus bias the device 340 towards the closed configuration.

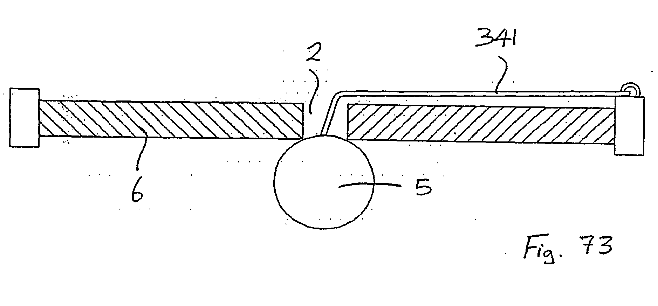

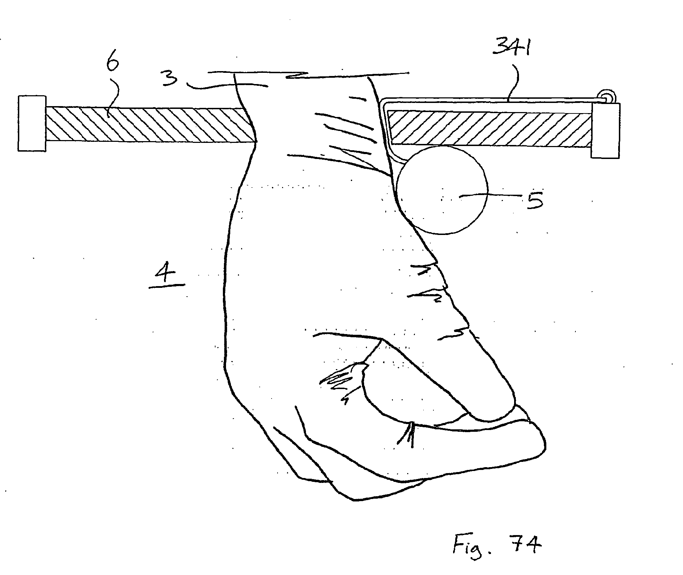

It will be appreciated that the first sealing member 5 may be attached to the second sealing member 6 in a variety of possible arrangements. For example, a single resilient cord 341 may extend from the sphere 5 through the passageway 2 and be attached to the proximal side of the second sealing member 6, as illustrated in Figs. 73 and 74.

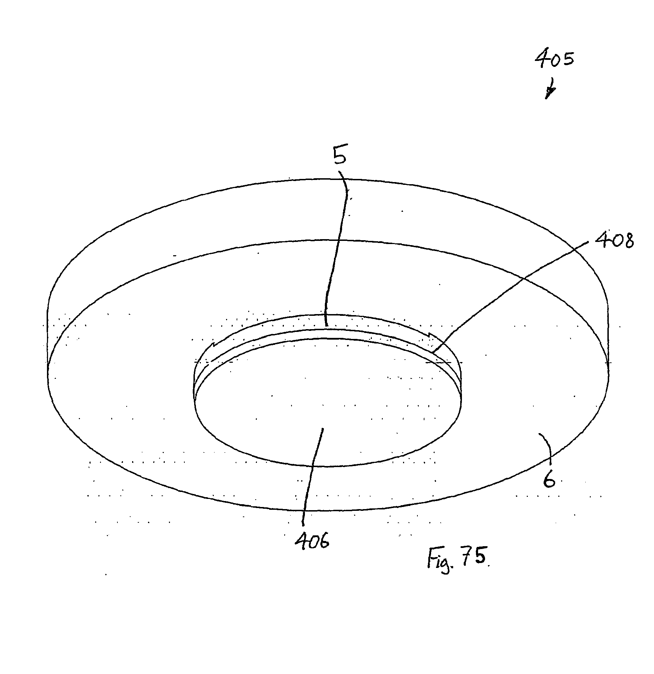

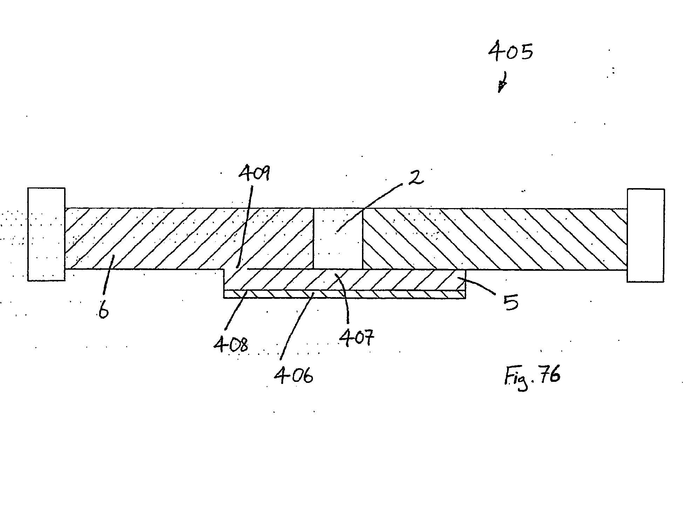

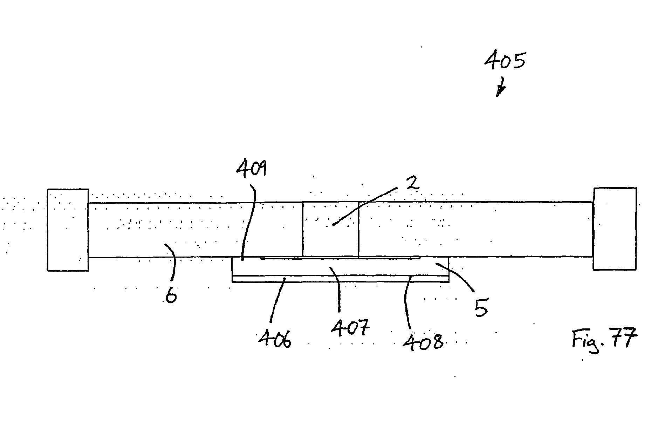

In Figs. 75 to 78 there is illustrated a surgical sealing device 405 according to the invention, which is similar to the device 1 of Figs. 1 to 8, and similar elements in Figs. 75 to 78 are assigned the same reference numerals.

The device 405 may be used in a laparoscopic procedure to effect a seal around a laparoscopic instrument inserted through the device 405 into an abdomen to maintain insufflation gas pressure within the abdomen. Alternatively, the device 405 may be used in a hand-assisted laparoscopic procedure to effect a seal around a surgeon's hand or forearm inserted through the device 405 into the abdomen to maintain insufflation gas pressure within the abdomen.

In this case the device 405 comprises a planar support element 406 fixedly attached to the proximal exterior surface 408 of the first sealing member 5. The support element 406 may be provided by any suitable material, such as fabric or plastic or rubber or metal. The support element 406 may be attached to the first sealing member 5 by any suitable means, such as by heat-sealing or by adhesive-backing or by gluing.

As discussed previously with reference to Figs. 1 to 8, the first sealing member 5 is fixedly attached to the second sealing member 6 at an attached region 409, with a detached region 407 remaining detached from the second sealing member 6.

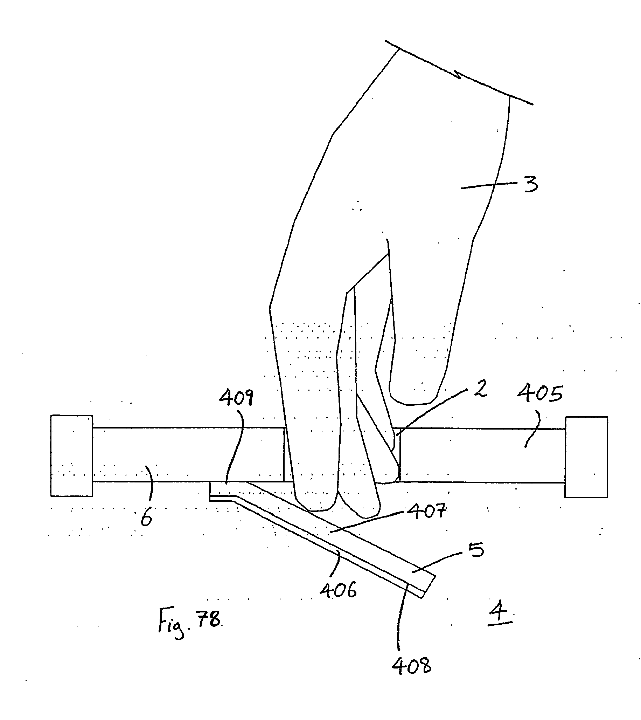

The support element 406 acts as a stiffening element to increase the stiffness of the detached region 407 of the first sealing member 5. By stiffening the detached region 407 of the first sealing member 5, the support element 406 minimises the deformation of the detached region 407 of the first sealing member 5 when the instrument or the surgeon's hand/forearm 3 is pushed through the passageway 2 (Fig. 78). In particular any deformation of the detached region 407 in the direction perpendicular to the plane of the first sealing member 5 is minimised.

Thus because there is a minimum of deformation of the first sealing member 5, it is easier for the surgeon to retract the first sealing member 5 laterally to reveal the distal end opening of the passageway 2 by pushing the instrument or the hand/forearm 3 through the passageway 2 (Fig. 78). It is therefore also easier for the surgeon to pass the instrument or the hand/forearm 3 through the device 405 and gain access to the abdomen 4 because of the support provided by the support element 406 to the first sealing member 5.

As illustrated in Fig. 78, when the surgeon pushes the hand/forearm 3 through the passageway 2, the fingers of the hand 3 engage the proximal side of the first sealing member 5. Further pushing of the hand/forearm 3 distally will cause the first sealing member 5 to be retracted laterally to reveal the distal end opening of the passageway 2, and without causing any substantial deformation of the detached region 407 of the first sealing member 5.

The attached region 409 of the first sealing member 5 is not stiffened by the support element 406, and the attached region 409 retains the required elasticity to facilitate retraction of the first sealing member 5 to reveal the distal end opening of the passageway 2.

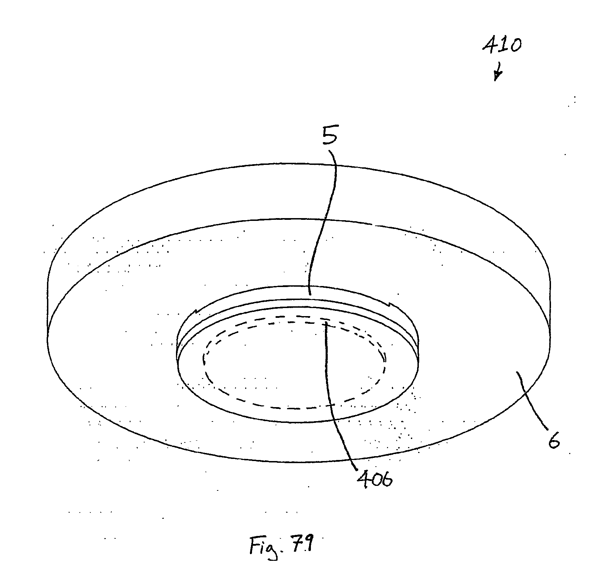

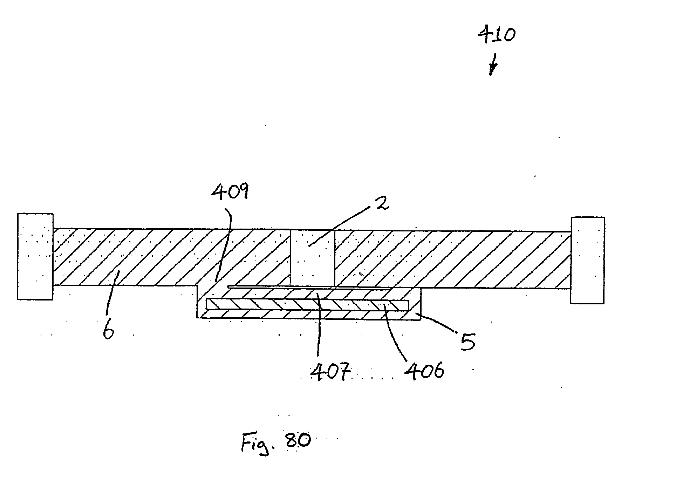

Figs. 79 and 80 illustrate another surgical sealing device 410 according to the invention, which is similar to the device 405 of Figs. 75 to 78, and similar elements in Figs. 79 and 80 are assigned the same reference numerals.

In this case, the support element 406 is embedded within the first sealing member 5, as illustrated in Fig. 80.

Because the support element 406 supports the detached region 407, this ensures that it is not difficult for the surgeon to retract the first sealing member 5 to reveal the distal end opening of the passageway 2 by inserting the instrument or the hand/forearm 3 through the passageway 2. In particular, the first sealing member 5 is supported and so does not deform distally downwardly perpendicular to the plane of the first sealing member 5 when the surgeon pushes the instrument or the hand/forearm 3 through the passageway 2. In such a manner it is relatively easy for the surgeon to access the abdomen 4 because the first sealing member 5 is prevented from deforming distally downwardly and instead is retracted to reveal the distal end opening of the passageway 2.

It will be appreciated that the support element feature as described above with reference to Figs. 75 to 80 may be employed with any of the surgical sealing devices described previously with reference to Figs. 1 to 74.

The invention is not limited to the embodiments hereinbefore described, with reference to the accompanying drawings, which may be varied in construction and detail.

## Claims

1. A surgical sealing device (405, 410) comprising:
a first sealing valve for sealing across an opening (2) to an internal cavity (4);
the first sealing valve comprising a first sealing member (5) and a second sealing member (6);
the sealing members (5, 6) being movable relative to one another between a closed configuration, in which the sealing members (5, 6) at least partially overlap one another, for sealing across the opening (2), and an open configuration for facilitating passage of an object through the first sealing valve to access the internal cavity (4);
the device comprising at least one support element (406) for at least partially supporting at least one of the sealing members (5, 6) to facilitate ease of passage of the object through the first sealing valve,
**characterised in that** the support element (406) is attached to the exterior surface (408) of the first sealing member (5) or **in that** the support element (406) is embedded within the first sealing member (5).

2. A device as claimed in claim 1 wherein the support element (406) is configured to at least partially support the sealing member (5, 6) to facilitate ease of movement of the sealing members (5, 6) relative to one another from the closed configuration to the open configuration.

3. A device as claimed in claim 1 or 2 wherein the support element (406) is configured to minimise deformation of at least part of the sealing member (5, 6).

4. A device as claimed in any of claims 1 to 3 wherein the sealing member (5, 6) is substantially planar.

5. A device as claimed in claim 4 wherein the support element (406) is configured to minimise deformation of at least part of the sealing member (5, 6) in a direction substantially perpendicular to the plane of the sealing member (5, 6).

6. A device as claimed in any of claims I to 5 wherein the support element (406) is substantially planar.

7. A device as claimed in any of claims 1 to 6 wherein the sealing members (5, 6) are fixedly attached to one another, each sealing member (5, 6) having a first region (409) attached to the other sealing member and a second region (407) which remains detached from the other sealing members (5, 6).

8. A device as claimed in claim 7 wherein the support element (406) is configured to minimise deformation of the second region of the sealing member.

9. A device as claimed in any of claims 3 to 8 wherein the support element (406) comprises a stiffening element (406) to increase the stiffness of at least part of the sealing member (5, 6).

10. A device as claimed in any of claims 1 to 9 wherein at least one of the sealing members (5, 6) is at least partially of a gelatinous elastomeric material.

11. A device as claimed in any of claims 1 to 10 wherein the first sealing valve is biased towards the closed configuration.

12. A device as claimed in any of claims 1 to 11 wherein the first sealing member (5) and the second sealing member (6) define overlapping portions, which overlap with each other in the closed configuration.

13. A device as claimed in any of claims 1 to 12 wherein a thin slit (407) is defined between the overlap of the first sealing member (5) over the second sealing member (6).

14. A device as claimed in any of claims 1 to 13 wherein the first sealing member (5) is fixedly attached to the second sealing member (6).

15. A device as claimed in any of claims 1 to 14 wherein the first sealing valve is configured to be located externally of an internal cavity proximally of an opening to the internal cavity.

16. A device as claimed in any of claims 1 to 14 wherein the first sealing valve is configured to be located within an internal cavity distally of an opening to the internal cavity.

17. A device as claimed in any of claims 1 to 16 wherein the device comprises a second sealing valve for sealing around an object passed through an opening to an internal cavity,

18. A device as claimed in claim 17 wherein the first sealing valve is located distally of the second sealing valve.

19. A device as claimed in claim 17 wherein the first sealing valve is located proximally of the second sealing valve.

20. A device as claimed in any of claims 17 to 19 wherein the first sealing valve is longitudinally spaced apart from the second sealing valve.

21. A device as claimed in any of claims 17 to 19 wherein the first sealing valve is located adjacent to and in close proximity to the second seal ing valve.

22. A device as claimed in any of claims 17 to 21 wherein the second sealing member provides the second sealing valve.

23. A device as claimed in any of claims 17 to 22 wherein the second sealing valve comprises a lip seal valve.

24. A device as claimed in any of claims 1 to 23 wherein the device (405, 410) is mountable to a retractor device (27).

25. A device us claimed in any of claims 1 to 24 wherein the first sealing member is hingeably movable relative to the second sealing member between the closed configuration and the open configuration.

26. A device as claimed in any of claims 1 to 25 wherein the first sealing member is at least partially curved.

27. A device as claimed in any of claims 1 to 26 wherein the second sealing member is substantially curved.

28. A device as claimed in any of claims 17 to 21 wherein the second sealing valve comprises a third sealing member having a passageway extending therethrough, through which an object may be inserted to access an internal cavity.

29. A device as claimed in any of claims 17 to 21 wherein the second sealing valve is at least partially inflatable.

30. A device as claimed in any of claims 17 to 21 wherein the second sealing valve comprises an iris valve.

31. A device as claimed in any of claims 1 to 30 wherein the device comprises a third sealing valve for sealing across an opening to an internal cavity.

32. A surgical assembly comprising:
a retractor device (22) for retracting the sides of an opening to an internal cavity; and
a surgical sealing device (405, 410) as claimed in any of claims I to 31.

33. An assembly as claimed in claim 32 wherein the surgical sealing device (405, 410) is mounted to the retractor device (27).

## Patentansprüche

1. Chirurgische Dichtungsvorrichtung (405, 410), die Folgendes umfasst:
ein erstes Dichtungsventil zum Dichten über einer Öffnung (2) zu einem inneren Hohlraum (4);
wobei das erste Dichtungsventil ein erstes Dichtungsglied (5) und ein zweites Dichtungsglied (6) umfasst;
wobei die Dichtungsglieder (5, 6) zwischen einer geschlossenen Konfiguration, zum Dichten über der Öffnung (2), in der die Dichtungsglieder (5, 6) einander mindestens teilweise überlappen, und einer offenen Konfiguration zum Ermöglichen des Hindurchgelangens eines Gegenstandes durch das erste Dichtungsventil, um Zugang zum inneren Hohlraum (4) zu erlangen, relativ zueinander beweglich sind;
wobei die Vorrichtung mindestens ein Stützelement (406) zum mindestens teilweisen Stützen mindestens eines der Dichtungsglieder (5, 6) umfasst, um das Hindurchgelangen des Gegenstands durch das erste Dichtungsventil zu erleichtern;
**dadurch gekennzeichnet, dass** das Stützelement (406) an der Außenseite (408) des ersten Dichtungsglieds (5) angebracht ist oder dass das Stützelement (406) im ersten Dichtungsglied (5) eingebettet ist.

2. Vorrichtung nach Anspruch 1, wobei das Stützelement (406) dazu ausgebildet ist, das Dichtungsglied (5, 6) mindestens teilweise zu stützen, um die Bewegung der Dichtungsglieder (5, 6) relativ zueinander aus der geschlossenen Konfiguration in die offene Konfiguration zu erleichtern.

3. Vorrichtung nach Anspruch 1 oder 2, wobei das Stützelement (406) dazu ausgebildet ist, die Verformung mindestens eines Teils des Dichtungsglieds (5, 6) zu minimieren.

4. Vorrichtung nach einem der Ansprüche 1 bis 3, wobei das Dichtungsglied (5, 6) im Wesentlichen eben ist.

5. Vorrichtung nach Anspruch 4, wobei das Stützelement (406) dazu ausgebildet ist, die Verformung mindestens eines Teils des Dichtungsglieds (5, 6) in einer zur Ebene des Dichtungsglieds (5, 6) im Wesentlichen senkrechten Richtung zu minimieren.

6. Vorrichtung nach einem der Ansprüche 1 bis 5, wobei das Stützelement (406) im Wesentlichen eben ist.

7. Vorrichtung nach einem der Ansprüche 1 bis 6, wobei die Dichtungsglieder (5, 6) fest aneinander angebracht sind, wobei die Dichtungsglieder (5, 6) jeweils eine erste Region (409), die am anderen Dichtungsglied angebracht ist, und eine zweite Region (407) haben, die von den anderen Dichtungsgliedern (5, 6) abgelöst bleibt.

8. Vorrichtung nach Anspruch 7, wobei das Stützelement (406) dazu ausgebildet ist, die Verformung der zweiten Region des Dichtungsglieds zu minimieren.

9. Vorrichtung nach einem der Ansprüche 3 bis 8, wobei das Stützelement (406) ein Versteifungselement (406) umfasst, um die Steifigkeit mindestens eines Teils des Dichtungsglieds (5, 6) zu erhöhen.

10. Vorrichtung nach einem der Ansprüche 1 bis 9, wobei mindestens eines der Dichtungsglieder (5, 6) mindestens teilweise aus einem gelatinösen Elastomermaterial besteht.

11. Vorrichtung nach einem der Ansprüche 1 bis 10, wobei das erste Dichtungsventil zu der geschlossenen Konfiguration hin vorgespannt ist.

12. Vorrichtung nach einem der Ansprüche 1 bis 11, wobei das erste Dichtungsglied (5) und das zweite Dichtungsglied (6) überlappende Abschnitte definieren, die in der geschlossenen Konfiguration einander überlappen.

13. Vorrichtung nach einem der Ansprüche 1 bis 12, wobei ein dünner Schlitz (407) zwischen der Überlappung des ersten Dichtungsglieds (5) über dem zweiten Dichtungsglied (6) definiert ist.

14. Vorrichtung nach einem der Ansprüche 1 bis 13, wobei das erste Dichtungsglied (5) fest am zweiten Dichtungsglied (6) angebracht ist.

15. Vorrichtung nach einem der Ansprüche 1 bis 14, wobei das erste Dichtungsventil dazu ausgebildet ist, außerhalb von einem inneren Hohlraum proximal zu einer Öffnung zum inneren Hohlraum angeordnet zu werden.

16. Vorrichtung nach einem der Ansprüche 1 bis 14, wobei das erste Dichtungsventil dazu ausgebildet ist, innerhalb von einem inneren Hohlraum distal von einer Öffnung zum inneren Hohlraum angeordnet zu werden.

17. Vorrichtung nach einem der Ansprüche 1 bis 16, wobei die Vorrichtung ein zweites Dichtungsventil zum Dichten um einen durch eine Öffnung zu einem inneren Hohlraum gesteckten Gegenstand umfasst.

18. Vorrichtung nach Anspruch 17, wobei das erste Dichtungsventil distal vom zweiten Dichtungsventil angeordnet ist.

19. Vorrichtung nach Anspruch 17, wobei das erste Dichtungsventil proximal zum zweiten Dichtungsventil angeordnet ist.

20. Vorrichtung nach einem der Ansprüche 17 bis 19, wobei das erste Dichtungsventil in Längsrichtung vom zweiten Dichtungsventil beabstandet ist.

21. Vorrichtung nach einem der Ansprüche 17 bis 19, wobei das erste Dichtungsventil neben dem und in unmittelbarer Nähe zum zweiten Dichtungsventil angeordnet ist.

22. Vorrichtung nach einem der Ansprüche 17 bis 21, wobei das zweite Dichtungsglied das zweite Dichtungsventil bereitstellt.

23. Vorrichtung nach einem der Ansprüche 17 bis 22, wobei das zweite Dichtungsventil ein Lippendichtungsventil umfasst.

24. Vorrichtung nach einem der Ansprüche 1 bis 23, wobei die Vorrichtung (405, 410) an einer Spreizervorrichtung (27) montierbar ist.

25. Vorrichtung nach einem der Ansprüche 1 bis 24, wobei das erste Dichtungsglied relativ zum zweiten Dichtungsglied gelenkig zwischen der geschlossenen Konfiguration und der offenen Konfiguration beweglich ist.

26. Vorrichtung nach einem der Ansprüche 1 bis 25, wobei das erste Dichtungsglied mindestens teilweise gekrümmt ist.

27. Vorrichtung nach einem der Ansprüche 1 bis 26, wobei das zweite Dichtungsglied im Wesentlichen gekrümmt ist.

28. Vorrichtung nach einem der Ansprüche 17 bis 21, wobei das zweite Dichtungsventil ein drittes Dichtungsglied mit einem sich dadurch hindurch erstreckenden Durchgang, durch den ein Gegenstand eingeführt werden kann, um Zugang zu einem inneren Hohlraum zu erlangen, umfasst.

29. Vorrichtung nach einem der Ansprüche 17 bis 21, wobei das zweite Dichtungsventil mindestens teilweise aufblasbar ist.

30. Vorrichtung nach einem der Ansprüche 17 bis 21, wobei das zweite Dichtungsventil ein Blendenventil umfasst.

31. Vorrichtung nach einem der Ansprüche 1 bis 30, wobei die Vorrichtung ein drittes Dichtungsventil zum Dichten über einer Öffnung zu einem inneren Hohlraum umfasst.

32. Chirurgische Montagegruppe, die Folgendes umfasst:
eine Spreizervorrichtung (22) zum Spreizen der Seiten einer Öffnung zu einem inneren Hohlraum; und
eine chirurgische Dichtungsvorrichtung (405, 410) nach einem der Ansprüche 1 bis 31.

33. Montagegruppe nach Anspruch 32, wobei die chirurgische Dichtungsvorrichtung (405, 410) an der Spreizervorrichtung (27) montiert ist.

## Revendications

1. Dispositif d'étanchéité chirurgical (405, 410) comprenant :
une première valve d'étanchéité pour obturer une ouverture (2) d'une cavité interne (4) ;
la première valve d'étanchéité comprenant un premier organe d'étanchéité (5) et un deuxième organe d'étanchéité (6) ;
les organes d'étanchéité (5, 6) étant mobiles l'un par rapport à l'autre entre une configuration fermée, où les organes d'étanchéité (5, 6) se recouvrent mutuellement au moins en partie, pour obturer l'ouverture (2), et une configuration ouverte pour faciliter le passage d'un objet à travers la première valve d'étanchéité afin d'avoir accès à la cavité interne (4) ;
le dispositif comprenant au moins un élément de soutien (406) pour soutenir au moins en partie au moins un des organes d'étanchéité (5, 6) afin de faciliter le passage de l'objet à travers la première valve d'étanchéité,
**caractérisé en ce que** l'élément de soutien (406) est attaché à la surface extérieure (408) du premier organe d'étanchéité (5) ou **en ce que** l'élément de soutien (406) est encastré à l'intérieur du premier organe d'étanchéité (5).

2. Dispositif conforme à la revendication 1, où l'élément de soutien (406) est configuré pour soutenir au moins en partie l'organe d'étanchéité (5, 6) afin de faciliter le mouvement des organes d'étanchéité (5, 6) l'un par rapport à l'autre depuis la configuration fermée jusqu'à la configuration ouverte.

3. Dispositif conforme à la revendication 1 ou 2, où l'élément de soutien (406) est configuré pour minimiser la déformation d'au moins une partie de l'organe d'étanchéité (5, 6).

4. Dispositif conforme à une quelconque des revendications 1 à 3, où l'organe d'étanchéité (5, 6) est sensiblement planaire.

5. Dispositif conforme à la revendication 4, où l'élément de soutien (406) est configuré pour minimiser la déformation d'au moins une partie de l'organe d'étanchéité (5, 6) dans une direction sensiblement perpendiculaire au plan de l'organe d'étanchéité (5, 6).

6. Dispositif conforme à une quelconque des revendications 1 à 5, où l'élément de soutien (406) est sensiblement planaire.

7. Dispositif conforme à une quelconque des revendications 1 à 6, où les organes d'étanchéité (5, 6) sont attachés de manière fixe l'un à l'autre, chaque organe d'étanchéité (5, 6) étant doté d'une première région (409) attachée à l'autre organe d'étanchéité et d'une deuxième région (407) qui reste détachée des autres organes d'étanchéité (5, 6).

8. Dispositif conforme à la revendication 7, où l'élément de soutien (406) est configuré pour minimiser la déformation de la deuxième région de l'organe d'étanchéité.

9. Dispositif conforme à une quelconque des revendications 3 à 8, où l'élément de soutien (406) comprend un élément raidisseur (406) pour augmenter la rigidité d'au moins une partie de l'organe d'étanchéité (5, 6).

10. Dispositif conforme à une quelconque des revendications 1 à 9, où au moins un des organes d'étanchéité (5, 6) est au moins en partie en élastomère gélatineux.

11. Dispositif conforme à une quelconque des revendications 1 à 10, où la première valve d'étanchéité est sollicitée vers la configuration fermée.

12. Dispositif conforme à une quelconque des revendications 1 à 11, où le premier organe d'étanchéité (5) et le deuxième organe d'étanchéité (6) délimitent des parties chevauchantes, qui se chevauchent mutuellement en configuration fermée.

13. Dispositif conforme à une quelconque des revendications 1 à 12, où une fente mince (407) est délimitée entre le chevauchement du premier organe d'étanchéité (5) au-dessus du deuxième organe d'étanchéité.

14. Dispositif conforme à une quelconque des revendications 1 à 13, où le premier organe d'étanchéité (5) est attaché de manière fixe au deuxième organe d'étanchéité (6).

15. Dispositif conforme à une quelconque des revendications 1 à 14, où la première valve d'étanchéité est configurée pour être située à l'extérieur d'une cavité interne, en position proximale par rapport à une ouverture de la cavité interne.

16. Dispositif conforme à une quelconque des revendications 1 à 14, où la première valve d'étanchéité est configurée pour être située à l'intérieur d'une cavité interne, en position distale par rapport à une ouverture de la cavité interne.

17. Dispositif conforme à une quelconque des revendications 1 à 16, où le dispositif comprend une deuxième valve d'étanchéité pour assurer l'étanchéité autour d'un objet passant à travers une ouverture d'une cavité interne.

18. Dispositif conforme à la revendication 17, où la première valve d'étanchéité est située en position distale par rapport à la deuxième valve d'étanchéité.

19. Dispositif conforme à la revendication 17, où la première valve d'étanchéité est située en position proximale par rapport à la deuxième valve d'étanchéité.

20. Dispositif conforme à une quelconque des revendications 17 à 19, où la première valve d'étanchéité est espacée longitudinalement de la deuxième valve d'étanchéité.

21. Dispositif conforme à une quelconque des revendications 17 à 19, où la première valve d'étanchéité est située en position adjacente et à proximité immédiate de la deuxième valve d'étanchéité.

22. Dispositif conforme à une quelconque des revendications 17 à 21, où le deuxième organe d'étanchéité sert de deuxième valve d'étanchéité.

23. Dispositif conforme à une quelconque des revendications 17 à 22, où la deuxième valve d'étanchéité comprend une valve à joint à lèvre.

24. Dispositif conforme à une quelconque des revendications 1 à 23, où le dispositif (405, 410) peut se monter sur un dispositif de type écarteur (27).

25. Dispositif conforme à une quelconque des revendications 1 à 24, où le premier organe d'étanchéité est mobile par articulation par rapport au deuxième organe d'étanchéité entre la configuration fermée et la configuration ouverte.

26. Dispositif conforme à une quelconque des revendications 1 à 25, où le premier organe d'étanchéité est courbe au moins en partie.

27. Dispositif conforme à une quelconque des revendications 1 à 26, où le deuxième organe d'étanchéité est sensiblement courbe.

28. Dispositif conforme à une quelconque des revendications 17 à 21, où la deuxième valve d'étanchéité comprend un troisième organe d'étanchéité muni d'un passage s'étendant en son travers, à travers lequel un objet peut être inséré pour avoir accès à une cavité interne.

29. Dispositif conforme à une quelconque des revendications 17 à 21, où la deuxième valve d'étanchéité est gonflable au moins en partie.

30. Dispositif conforme à une quelconque des revendications 17 à 21, où la deuxième valve d'étanchéité comprend une valve à diaphragme iris.

31. Dispositif conforme à une quelconque des revendications 1 à 30, où le dispositif comprend une troisième valve d'étanchéité pour obturer une ouverture d'une cavité interne.

32. Ensemble chirurgical comprenant :
un dispositif de type écarteur (22) pour écarter les bords d'une ouverture d'une cavité interne ; et
un dispositif d'étanchéité chirurgical (405, 410) conforme à une quelconque des revendications 1 à 31.

33. Ensemble conforme à la revendication 32, où le dispositif d'étanchéité chirurgical (405, 410) est monté sur le dispositif de type écarteur (27).
